# EUROPEAN PATENT APPLICATION

(11) **EP 4 837 963 A1**
(43) Date of publication of application: **11.11.2026**
(21) Application number: 24915750.4
(22) Date of filing: 12.01.2024
(51) Int. Cl.: C12N 15/113, A61K 39/00

(54) **NUCLEIC ACID MOLECULE WITH HAIRPIN STRUCTURE CAPABLE OF MODULATING INNATE IMMUNITY AND USE THEREOF**

(30) Priority: 02.01.2024 KR 20240000394
(71) Applicant: Korea Advanced Institute of Science and Technology, Daejeon 34141 (KR); Korea National Institute of Health, Cheongju-si, Chungcheongbuk-do 28159 (KR)
(72) Inventor: KIM, Yoosik, Daejeon 34141 (KR); LEE, Keonyong, Daejeon 34141 (KR); KIM, Soo Hwan, Daejeon 34141 (KR); YEO, Jinah, Cheongju-si Chungcheongbuk-do 28159 (KR); LEE, Taeyoung, Cheongju-si Chungcheongbuk-do 28159 (KR); JEON, Ji Hyang, Cheongju-si Chungcheongbuk-do 28159 (KR); KIM, Dokeun, Cheongju-si Chungcheongbuk-do 28159 (KR); KIM, You-Jin, Cheongju-si Chungcheongbuk-do 28159 (KR); KIM, Seo Yeon, Cheongju-si Chungcheongbuk-do 28159 (KR)
(74) Representative: Mewburn Ellis LLP
(86) International application number: PCT/KR2024/000633
(87) International publication number: WO 2025/146858

(57) **Abstract**

The present invention relates to a nucleic acid molecule capable of controlling innate immunity by RNA and, more specifically, to a short duplex hairpin RNA (sdhRNA) that binds to protein kinase R (PKR), which is activated upon recognition of RNA, thereby inhibiting the activity of PKR and suppressing PKR-mediated innate immunity and which, when introduced into the 3'-UTR of mRNA, can increase the expression level of an antigen. The sdhRNA motif according to the present invention inhibits the activity of protein kinase R (PKR) by binding to PKR activated by recognizing RNA. Therefore, when administered into cells in combination with exogenous RNA or as an insert into the 3'-UTR of mRNA, the sdhRNA motif exhibits the effect of reducing RNA-induced PKR activation, mitigating RNA-induced cell death, and downregulating the expression of interferon-stimulated gene (ISG) as well as improving the expression efficiency of a protein encoded by the mRNA. With the effects, the motif is applied to various RNA-based uses, for example, vaccines, in vivo/ex vivo gene therapeutic agents, and the like.

## Description

### [Technical Field]

The present invention relates to a nucleic acid molecule capable of regulating RNA-mediated innate immune activation, and more particularly, to a short duplex hairpin RNA (sdhRNA) that binds to protein kinase R (PKR), which is activated upon recognition of RNA, thereby inhibiting PKR activation to suppress PKR-mediated innate immunity, and capable of increasing the expression level of an antigen when introduced into the 3'-UTR of mRNA.

### [Background Art]

With advances in molecular biology, nucleic acid (DNA/mRNA) vaccines using plasmids containing genes encoding protein/peptide antigens that elicit immune responses have been developed. Although nucleic acid vaccines were proposed in the early 1990s as a powerful alternative to conventional vaccines, they were not practically utilized until the COVID-19 pandemic due to limitations such as the low stability of mRNA and inefficient *in vivo* delivery. Over the past decade, technological innovations and research and development investments have positioned mRNA as a promising therapeutic tool in vaccine development and protein replacement therapies. mRNA vaccines offer numerous advantages over DNA vaccines as well as subunit, inactivated, and live-attenuated viral vaccines.

The primary advantage of mRNA vaccines is that, since host cells produce the antigen, traditional manufacturing processes that require culturing pathogens and isolating components for vaccine use are not necessary. In addition, whereas conventional vaccines predominantly activate humoral immunity, mRNA vaccines also induce cell-mediated immune responses during intracellular antigen production. Furthermore, mRNA vaccines are non-infectious and non-integrating platforms, and thus present a low potential risk of infection or mutagenesis due to insertion into human DNA and elicit minimal unnecessary immune responses. mRNA vaccines also enable rapid uptake and expression within the cytoplasm and, due to reduced immune responses compared to viral delivery methods, may be administered repeatedly.

mRNA vaccines are produced by *in vitro* transcription (IVT), enabling rapid, cost-effective, and large-scale production due to high yields. Moreover, mRNA introduced into cells may generate proteins at levels exceeding 100- to 1,000-fold, such that even small amounts of delivered mRNA may be effective.

However, RNA intended for use in gene therapy, including mRNA vaccines, is recognized as exogenous RNA within cells, triggering innate immune responses. Accordingly, there is a need to develop technologies capable of regulating innate immune activation induced by therapeutic or vaccine mRNA. In general, mRNA is composed of a cap structure, 5'- and 3'-untranslated regions (UTRs), an open reading frame (ORF), and a poly(A) tail, and optimization of nucleic acid constructs may enhance protein (antigen) expression efficiency, increase *in vivo* immunogenicity, and minimize unnecessary immune responses.

Various means and methods have been disclosed for increasing mRNA stability, reducing immunogenic responses triggered by mRNA administered to cells or organisms, and enhancing expression efficiency (i.e., transcriptional and/or translational efficiency) (US 10080809, US 2018-0353618, and US 2019-0144883). However, there remains a need for improved additional or alternative means, particularly for increasing expression efficiency (i.e., transcriptional and/or translational efficiency), because expression efficiency is an essential parameter for the intended medical applications, as it determines, for example, the dosage and dosing interval of mRNA therapeutics and ultimately governs the bioavailability of the final product, namely the encoded peptide or protein. At the same time, there remains a continuing need to further reduce manufacturing costs of mRNA therapeutics, increase the yield of produced mRNA molecules, and expand the available space within produced mRNA molecules for the actual transgene, namely the coding region encoding the desired polypeptide.

Numerous challenges remain in connection with nucleic acid delivery to elicit desired responses in biological systems. Although nucleic acid-based therapeutics such as vaccines hold significant promise, there remains a need for more efficient delivery of nucleic acids to appropriate sites within cells or organisms to realize this potential.

However, the use of nucleic acids for therapeutic and prophylactic purposes currently faces two major challenges. First, free RNA is susceptible to nuclease degradation in plasma. Second, free RNA has limited ability to access intracellular compartments in which the relevant translational machinery resides. Lipid nanoparticles formed from cationic lipids and other lipid components such as neutral lipids, cholesterol, PEG, PEGylated lipids, and oligonucleotides have been explored to prevent RNA degradation in plasma and to promote cellular uptake of nucleic acids.

Accordingly, the present inventors have made great efforts to solve the above-described problems and to develop a nucleic acid construct capable of suppressing innate immune responses and enhancing translational efficiency, and ascertained that, when a short duplex hairpin RNA molecule that inhibits PKR protein activation is selected and introduced into cells, innate immune responses may be suppressed and translational efficiency may be enhanced, thus culminating in the present invention.

### [Disclosure]

An object of the present invention is to provide a short duplex hairpin nucleic acid capable of suppressing innate immunity and enhancing translational efficiency.

Another object of the present invention is to provide a nucleic acid construct including the short duplex hairpin nucleic acid.

Still another object of the present invention is to provide a vaccine composition including the nucleic acid construct.

In order to accomplish the above objects, the present invention provides a short duplex hairpin nucleic acid (sdhNA) represented by a nucleotide sequence of any one of SEQ ID NOs: 1 to 8, which suppresses innate immune responses.

The present invention also provides a nucleic acid construct including, in the 5' to 3' direction: (a) at least one coding region; (b) a 3'-untranslated region (3'-UTR); (c) the short duplex hairpin nucleic acid (sdhNA); and (d) a poly(A) tail of 10 to 1000 nucleotides or a poly(A) tail-like sequence.

The present invention also provides a vaccine composition including the nucleic acid construct.

The present invention also provides the use of the vaccine composition for the prevention of a disease.

The present invention also provides a method of preventing a disease, including administering the vaccine composition.

The present invention also provides the use of the vaccine composition for the manufacture of a medicament for the prevention of a disease.

### [Brief description of Drawings]

FIG. 1 is a schematic diagram showing suppression of innate immune responses induced by mRNA administration and enhancement of antigen expression by sdhRNA of the present invention;

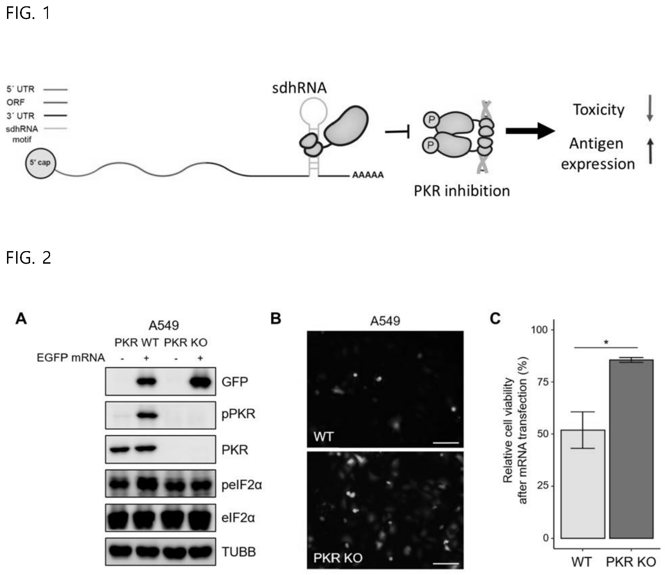

FIG. 2 shows analysis of antigen expression from EGFP mRNA administered to PKR-deleted A549 lung cancer cells, as well as cell death induced by exogenous mRNA delivery, (A) showing results of analysis of EGFP expression, PKR activation (pPKR), and p-eIF2α as a substrate of PKR by Western blotting in A549 cells (PKR WT) and PKR-deleted A549 cells (PKR KO), in which TUBB was used as a loading control, (B) showing results of analysis of EGFP fluorescence by fluorescence microscopy after administration of EGFP mRNA to A549 WT and PKR KO A549 cells, and (C) showing results of analysis of cell death in the two cell lines after administration of EGFP mRNA;

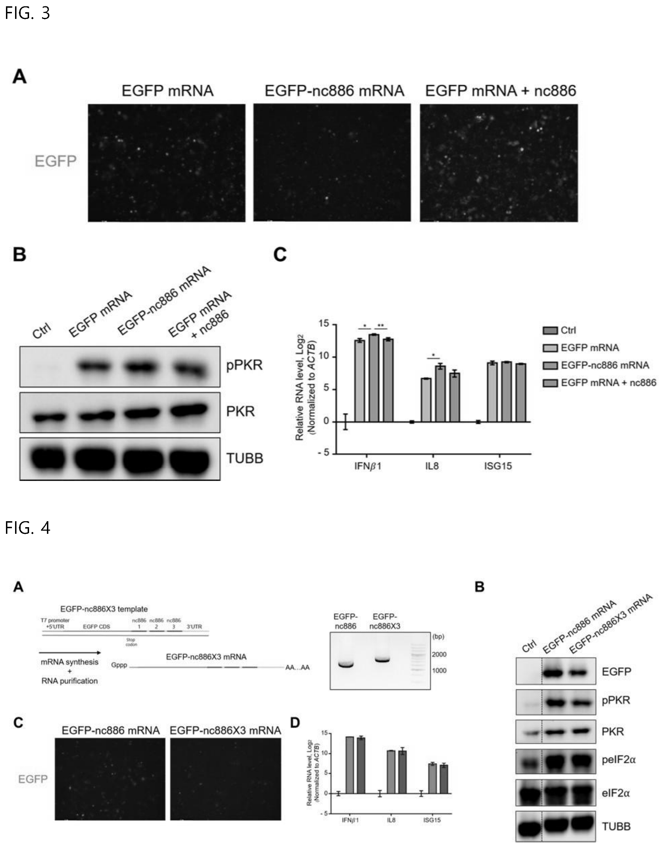

FIG. 3 shows results of analysis of antigen expression and interferon responses when nc886 RNA was administered to A549 cells either in the 3' UTR of EGFP mRNA or together with EGFP mRNA, (A) showing results of measurement of EGFP fluorescence when cells were treated with EGFP mRNA, EGFP mRNA containing the nc886 sequence in the 3' UTR, or EGFP mRNA and nc886 administered separately in combination, (B) showing results of analysis of PKR activation (pPKR) by Western blotting, in which TUBB was used as a loading control, and (C) showing results of measurement of expression levels of IFNβ1, IL8, and ISG15 mRNA by RT-qPCR;
FIG. 4 shows results of inserting three nc886 sequences into the 3' UTR of EGFP and analyzing the effects thereof, (A) showing an experimental scheme and EGFP mRNA synthesis results, (B) showing results of analysis of EGFP expression and PKR activation after administration of EGFP mRNA containing a single nc886 sequence and EGFP mRNA containing three nc886 sequences to A549 cells, (C) showing results of analysis of EGFP fluorescence of each construct, and (D) showing results of analysis of representative interferon genes IFNβ1, IL8, and ISG15 mRNA by RT-qPCR;
FIG. 5 shows results of analysis of PKR binding affinity of 1,840 hairpin sdhRNAs constructed by IVT, in which 1,840 hairpin RNAs were incubated with Flag-PKR obtained from HEK-293T cells, followed by immunoprecipitation, and the resulting PKR-sdhRNA complexes were analyzed by RNA-seq along with input RNA from immunoprecipitation, and PKR binding affinity for each RNA was determined by dividing the number of sequencing reads obtained from PKR immunoprecipitation by the number of input RNA sequencing reads;
FIG. 6 shows results of evaluation of PKR activation in cells after administration of poly(I:C), which induces PKR activation, following administration of seven sdhRNAs having different PKR binding affinities to A549 cells;
FIG. 7 shows results of analysis of PKR activation after co-administration of poly(I:C) with nc886, sdhRNA_mid_1, and sdhRNA _mid_2 to A549 cells;
FIG. 8 shows a schematic diagram of synthesis of EGFP mRNA incorporating nc886, sdhRNA_mid_1, sdhRNA_mid_2, and sdhRNA_ctrl in the 3' UTR, and results confirming mRNA synthesis by electrophoresis;
FIG. 9 shows results of analysis of PKR activation after administration of the EGFP mRNA synthesized in FIG. 8 to A549 cells;
FIG. 10 shows results of RT-qPCR analysis of IFNB1 and CXCL10 mRNA in cells treated with the four types of EGFP mRNA in FIG. 9;
FIG. 11 shows results of analysis of EGFP expression and cell death in A549 cells treated with the four types of EGFP mRNA used in FIG. 9, (A) showing results of analysis of EGFP protein fluorescence, (B) showing results of analysis of EGFP expression by Western blotting, and (C) showing results of analysis of cell death induced by EGFP mRNA;
FIG. 12 shows results of analysis of mRNA expression levels after administration of EGFP mRNA incorporating sdhRNA_mid_1, sdhRNA_mid_2, and sdhRNA_ctrl to A549 cells, in which the left panel shows changes in relative mRNA expression over time after cell administration, and the right panel shows the expression levels of mRNA remaining in cells after 24 hours;
FIG. 13 shows results of analysis of EGFP expression, PKR activation, and cell death after administration of EGFP mRNA incorporating nc886, sdhRNA_mid_1, sdhRNA_mid_2, and sdhRNA_ctrl to A549 cells using lipid nanoparticles, (A) showing an experimental scheme, (B) showing results of analysis of EGFP expression and PKR activation induced by EGFP mRNA incorporating sdhRNA by Western blotting, and (C) showing results of analysis of cell death induced by EGFP mRNA incorporating sdhRNA;
FIG. 14 shows results of analysis of PKR binding affinity after synthesizing a total of seven sdhRNAs and nc886 using either uridine or pseudouridine to evaluate the effect of pseudouridine;
FIG. 15 shows results of analysis of EGFP expression and PKR activation after synthesizing EGFP mRNA incorporating nc886, sdhRNA_mid_2, and sdhRNA_ctrl using either uridine or pseudouridine and administering the same to A549 cells, (A) showing results of analysis of EGFP expression and PKR activation by Western blotting, and (B) showing results of analysis of EGFP fluorescence by fluorescence microscopy;
FIG. 16 shows results of analysis of the effect on cell death of EGFP mRNA synthesized using uridine or pseudouridine in FIG. 15, in which the upper panel shows results of analysis of cleaved PARP, a cell death marker, by Western blotting, and the lower panel shows results of analysis of cell death by an MTT assay;
FIG. 17 shows results of analysis of Spike protein expression and PKR activation after inserting nc886, sdhRNA_ctrl, sdhRNA_mid_1, or sdhRNA _mid_2 into the 3' UTR of mRNA encoding the Spike protein of SARS-CoV-2 and administering the same to A549 cells;
FIG. 18 shows results of analysis of antigen-specific T cell numbers after inserting nc886, sdhRNA_mid_1, and sdhRNA _mid_2 into the 3' UTR of SARS-CoV-2 Spike mRNA, loading the same into lipid nanoparticles, and administering the formulation twice to mice; and
FIG. 19 shows results of analysis of antigen-specific antibodies after inserting nc886, sdhRNA_mid_1, and sdhRNA _mid_2 into the 3' UTR of SARS-CoV-2 Spike mRNA, loading the same into lipid nanoparticles, and administering the formulation twice to mice.

### [Mode for Invention]

Unless otherwise defined, all technical and scientific terms used herein have the same meanings as typically understood by those skilled in the art to which the present invention belongs. In general, the nomenclature used herein and the experimental methods described below are well known in the art and are typical.

In the present invention, it was investigated whether co-delivery of sdhRNA with exogenous RNA into cells may suppress the process in which RNA is recognized by innate immune response proteins such as protein kinase R (PKR), melanoma differentiation-associated gene 5 (MDA5), and retinoic acid-inducible gene I (RIG-I), thereby inducing immune responses, leading to cell death, inhibition of protein translation, and promotion of ISG expression, and whether expression levels may be increased.

In one embodiment of the present invention, PKR binding affinities of 1,840 hairpin sdhRNAs were analyzed, and sdhRNAs capable of inhibiting PKR activation and increasing expression of a target protein were selected (FIG. 1).

Accordingly, an aspect of the present invention relates to:
a short duplex hairpin nucleic acid (sdhNA) represented by a nucleotide sequence of any one of SEQ ID NOs: 1 to 8, which suppresses innate immune responses.

The present invention also relates to:
a nucleic acid construct including, in the 5' to 3' direction:
(a) at least one coding region;
(b) a 3'-untranslated region (3'-UTR);
(c) the short duplex hairpin nucleic acid; and
(d) a poly(A) tail of 10 to 1,000 nucleotides or a poly(A) tail-like sequence.

In the present invention, the short duplex hairpin nucleic acid (sdhNA) may be a short duplex hairpin RNA (sdhRNA).

In the present invention, the sdhRNA may be introduced into cells alone or in a form linked to the 3'-UTR of mRNA.

**[Table 1]**

| **SEQ ID NO**: | **sdhRNA** | **Sequence (5'** - **3')** |
|---|---|---|
| 1 | sdhRNA high 1 | GCCUAUUCAGUUACAGCGAGGUCUCAAUGCCUUCAUAGCCCUGUAC AAUGCUGCUUGAUCCAUAUGCAACAAGGCAGCACUGUAAAGAAGCC GAGGGCAGUAAGAAAACUUCCGUACUGAAGCUAGCAAC |
| 2 | sdhRNA high 2 | GCCUAUUCAGUUACAGCGUUCCUACUCAACUUCUCAUUUUCUGGUU CCUUCUACCAAGUUAAGCAUACUCAUUAGAAGGAACCAGAAAAUGA GAAGUUAAAUCUGAAGUCCACGUACUGAAGCUAGCAAC |
| 3 | sdhRNA high 3 | GCCUAUUCAGUUACAGCGUUUGCUGGCUGGUGCAGUGCCUGAGGGA GUAAGAGUCCUGUUGUUGUAAGAUAGUGUCUUACUCCCUCAGGCAC AUCUCCAACAAGUCUCUUGCCGUACUGAAGCUAGCAAC |
| 4 | sdhRNA high 4 | GCCUAUUCAGUUACAGCGUUAUUAACUCUGGCUUUUAACUUUGAUG GCAAAGGGGUAGCUAAACAAUCUAUGUCUUUGCCAUCAAAGUUAAA AGCCAUAGUUAAUUUCCUCCCGUACUGAAGCUAGCAAC |
| 5 | sdhRNA mid 1 | GCCUAUUCAGUUACAGCGAAGUUAUGCUUCUCCUGGGGUCUUCCUC UACAUUUCCACCUAGACGGGCCUGGGUCAAAAUGUAGAGGAAGGAC CCCAGAAGGAGCAAAACUUUCCGUACUGAAGCUAGCAAC |
| 6 | sdhRNA mid 2 | GCCUAUUCAGUUACAGCGGGCGCUUCGCUCCCCUCCGCCUUCUCUU CCCGGUUCUUCCCGGAGUCGGAAAAGCUGGGUUGAGAGGGCGAAA AAGGAUGAGGUGACUGGUCUCGUACUGAAGCUAGCAAC |
| 7 | sdhRNA _low 1 (Also sdhRNA ctrl) | GCCUAUUCAGUUACAGCGCAGUCAGAAUAAUGUCAAAGUGCUUACA GUGCAGGUAGUGAUAUGUGCAUCUACUGCAGUGAAGGCACUUGUAG CAUUAUGGUGACAGCUGCCUCGUACUGAAGCUAGCAAC |
| 8 | sdhRNA low 2 | GCCUAUUCAGUUACAGCGCUUUCCCAGGAGGGUGAAGAUGGUGCUG UGCUGAGGAAAGGGGAUGCAGAGCCCUGCCCAGCACCACCACCUCC UAUGCUCCUGGAUCCCUAGGCGUACUGAAGCUAGCAAC |
| 9 | nc886 | GCCUAUUCAGUUACAGCGCGGGUCGGAGUUAGCUCAAGCGGUUACC UCCUCAUGCCGGACUUUCUAUCUGUCCAUCUCUGUGCUGGGGUUCG AGACCCGCGGGUGCUUACUGACCCUUUUAUGCAACGUACUGAAGCU AGCAAC |
| 10 | Normalization RNA | GCCUAUUCAGUUACAGCGACCCCCAAAGCUCUCCUGCCUGCUUCUG UGUGAUAUGUUUGAUAUUGGGUUGUUUAAUUAGGAACCAACUAAAU GUCAAACAUAUUCUUACAGCAGCAGGUGAUUCAGCACCACCCUCUU UCAUACGUACUGAAGCUAGCAAC |

In the present invention, the nucleic acid construct may further include a 5'-cap structure and a 5'-UTR polynucleotide upstream of at least one coding region.

As used herein, the term "UTR" refers to an "untranslated region" located upstream (5') and/or downstream (3') of the coding region of a nucleic acid molecule described herein, and thus typically flanking the coding region. Accordingly, the term "UTR" generally includes a 3'-untranslated region ("3'-UTR") and a 5'-untranslated region ("5'-UTR"). A UTR may include or consist of a nucleic acid sequence that is not translated into a protein. Typically, a UTR includes "regulatory elements."

The term "regulatory element" refers to a nucleic acid sequence having gene regulatory activity, particularly the ability to influence expression including transcription or translation of an operably (in cis or in trans) linked transcribable nucleic acid sequence. The term includes promoters, enhancers, internal ribosome entry sites (IRES), introns, leaders, transcription termination signals such as polyadenylation signals and poly-U sequences, and other expression-regulating elements. Regulatory elements may function constitutively or in a temporal- and/or cell-specific manner. Optionally, regulatory elements may function through interaction (e.g., recruitment and binding) with regulatory proteins capable of modulating (e.g., inducing, enhancing, reducing, abolishing, or preventing) gene expression, particularly transcription.

Preferably, the UTR is operably linked to the coding region, namely arranged in a functional relationship so as to control (i.e., mediate or regulate, preferably enhance) expression of the coding sequence.

As used herein, the term "5'-UTR" refers to a portion of a nucleic acid molecule that is located 5' (i.e., upstream) of an open reading frame and is not translated into a protein. In the context of the present invention, the 5'-UTR begins at the transcription start site and terminates one nucleotide upstream of the start codon of the open reading frame.

The 5'-UTR may include elements that regulate gene expression, referred to as "regulatory elements." Such regulatory elements may, for example, be a ribosome binding site. The 5'-UTR may be modified by post-transcriptional modifications, such as addition of a 5'-cap. Accordingly, the 5'-UTR may correspond to a nucleic acid sequence located between the 5'-cap and the start codon, particularly a sequence of mature mRNA, more specifically, a sequence extending from a nucleotide located 3' to the 5'-cap, preferably a nucleotide located immediately downstream of the 5'-cap, to a nucleotide located 5' to the start codon of the protein-coding sequence (translation initiation site), preferably a nucleotide located immediately upstream of the start codon of the protein-coding sequence (translation initiation site).

The nucleotide located immediately downstream of the 5'-cap of mature mRNA typically corresponds to the transcription start site. The length of the 5'-UTR is generally less than 500, 400, 300, 250, or 200 nucleotides. In some embodiments, the length thereof may be at least 10, 20, 30, or 40 nucleotides and at most 50 nucleotides, preferably with a lower limit of 10 nucleotides.

In the present invention, the 5'-UTR polynucleotide may be selected from the group consisting of an α-globin 5'-UTR; an Hsp70 5'-UTR; an axon dynein heavy chain 2 (DNAH2) 5'-UTR; a hydroxysteroid dehydrogenase (3β-HSD) 5'-UTR; a 5'-UTR represented by the nucleotide sequence of SEQ ID NO: 11; and a 5'-UTR represented by the nucleotide sequence of SEQ ID NO: 12, but is not limited thereto.
SEQ ID NO: 11: 5'-UTR_EGFR
   5'-GGUUUAGUGAACCGUCAGAUCCGCUAGCGCUACCGGUCGCCACC-3'
SEQ ID NO: 12: 5'-UTR_Spike
   5'-GGAAAUAAGAGAGAAAAGAAGAGUAAGAAGAAAUAUAAGAGCCACC-3'

The 5'-cap of natural mRNA is associated with nuclear export, increases mRNA stability, and binds to mRNA cap-binding proteins (CBPs). Such binding results in the formation of a mature circularized mRNA species through association of poly (A) -binding proteins (PABPs) with CBPs, thereby contributing to mRNA stability in cells and during translation. The cap further facilitates removal of 5'-proximal introns during mRNA splicing.

In the present invention, the 5'-cap is typically a modified nucleotide (cap analog), particularly a guanine nucleotide added to the 5' end of an mRNA molecule. Preferably, the 5'-cap is added via a 5'-5' triphosphate linkage (also referred to as m7GpppN). Additional examples of 5'-cap structures include glycerol, inverted deoxy abasic residues (moieties), 4',5'-methylene nucleotides, 1-(β-D-erythrofuranosyl) nucleotides, 4'-thio nucleotides, carbocyclic nucleotides, 1,5-anhydrohexitol nucleotides, L-nucleotides, α-nucleotides, modified base nucleotides, threo-pentofuranosyl nucleotides, acyclic 3',4'-seco nucleotides, acyclic 3,4-dihydroxybutyl nucleotides, acyclic 3,5-dihydroxypentyl nucleotides, 3'-3' inverted nucleotide moieties, 3'-3' inverted abasic moieties, 3'-2' inverted nucleotide moieties, 3'-2' inverted abasic moieties, 1,4-butanediol phosphate, 3'-phosphoramidate, hexyl phosphate, aminohexyl phosphate, 3'-phosphate, 3'-phosphorothioate, phosphorodithioate, or bridging or non-bridging methylphosphonate moieties.

Such modified 5'-cap structures may be used to modify the mRNA sequence of the nucleic acid construct of the present invention.

Additional modified 5'-cap structures that may be used in the present invention include CAP1 (additional methylation of the ribose of the nucleotide adjacent to m7GpppN), CAP2 (additional methylation of the ribose of the second downstream nucleotide of m7GpppN), CAP3 (additional methylation of the ribose of the third downstream nucleotide of m7GpppN), CAP4 (additional methylation of the ribose of the fourth downstream nucleotide of m7GpppN), ARCA (anti-reverse cap analog), modified ARCA (e.g., phosphorothioate-modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

In the present invention, the 5'-cap structure may be formed by chemical RNA synthesis or by RNA *in vitro* transcription using a cap analog (co-transcriptional capping), or alternatively, the cap structure may be formed *in vitro* using capping enzymes (e.g., commercially available capping kits).

In the present invention, the cap analog is a non-polymerizable dinucleotide having cap functionality that promotes translation or localization and/or prevents degradation of an RNA molecule when introduced at the 5' end of the RNA molecule. "Non-polymerizable" means that, since the cap analog lacks a 5' triphosphate, it may only be incorporated at the 5' end and cannot be extended in the 3' direction by template-dependent RNA polymerases.

The cap analog includes, but is not limited to, a chemical structure selected from the group consisting of m7GpppA, m⁷GpppAₘpG, unmethylated cap analogs, dimethylated cap analogs, trimethylated cap analogs (e.g., m2,2,7GpppA), dimethylated symmetric cap analogs (e.g., m7Gpppm7A), and anti-reverse cap analogs (e.g., ARCA; m7,2'-OmeGpppA, m7,2'-dGpppA, m7,3'-OmeGpppA, m7,3'-dGpppA, and tetraphosphate derivatives thereof).

Additional cap analogs have been described previously (US 7,074,596, WO 2008/016473, WO 2008/157688, WO 2009/149253, WO 2011/015347, and WO 2013/059475).

In the present invention, the 5'-cap structure may be selected from the group consisting of anti-reverse cap analogs (m₂^{7,3'-O}G(5')ppp(5')G), but is not limited thereto.

In the present invention, the coding region may encode at least one protein selected from the group consisting of antigenic proteins, allergenic proteins, therapeutic proteins, and fragments, variants, or derivatives thereof, but is not limited thereto.

In the present invention, the antigenic protein may be at least one selected from the group consisting of tumor antigens, pathogenic antigens, autoantigens, alloantigens, and allergenic antigens, but is not limited thereto.

As used herein, the term "tumor antigen" refers to an antigenic (poly)peptide or protein derived from or associated with a (preferably malignant) tumor or cancer disease. The terms "cancer" and "tumor" are used interchangeably herein to refer to a neoplasm characterized by uncontrolled and generally rapid proliferation of cells that tend to invade surrounding tissues and metastasize to distant sites. These terms include both benign and malignant neoplasms. Malignant tumors are typically characterized by anaplasia, invasiveness, and metastasis, whereas benign tumors generally lack these characteristics. The terms "cancer" and "tumor" further include neoplasms characterized by tumor growth as well as cancers of the hematopoietic and lymphatic systems. Tumor antigens are typically derived from tumor/cancer cells, preferably mammalian tumor/cancer cells, and may be located within or on the surface of tumor cells, tumors, for example systemic or solid tumors, derived from mammals, preferably humans. Tumor antigens generally include tumor-specific antigens (TSAs) and tumor-associated antigens (TAAs). TSAs typically arise from tumor-specific mutations and are specifically expressed by tumor cells. Many common TAAs are generally presented by both tumor and "normal" (healthy, non-tumor) cells.

In the present invention, the antigen may be a tumor-associated protein or nucleic acid sequence, in which each nucleic acid sequence encodes a different peptide or protein, and the at least one nucleic acid sequence may encode 5T4, 707-AP, 9D7, AFP, AlbZIP HPG1, alpha-5-beta-1 integrin, alpha-5-beta-6 integrin, alpha-actinin-4/m, alpha-methylacyl-CoA racemase, AT-4, ARTC1/m, B7H4, BAGE-1, BCL-2, bcr/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CDE30, CD33, CD4, CD52, CD55, CD56, CD80, CDC27/m, CDK4/m, CDKN2A/m, CEA, CLCA2, CML28, CML66, COA-1/m, coactosin-like protein, collagen XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCAM, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERVK-MEL, HLA-A*0201-R17I, HLA-A11/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1R, IL-13Rα2, IL-2R, IL-5, immature laminin receptor, kallikrein-2, kallikrein-4, Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGED2, MAGE-D4, MAGE-E1, MAGE-E2, MAGE-F1, MAGE-H1, MAGEL2, mammaglobin A, MART-1/Melan-A, MART-2, MART-2/m, stromal protein 22, MC1R, M-CSF, ME1/m, mesothelin, MG50/PXDN, MMP11, MN/CA IX antigen, MRP-3, MUC-1, MUC-2, MUM-1/m, MUM-2/m, MUM-3/m, myosin class I/m, NA88-A, N-acetylglucosaminyltransferase-V, neo-PAP, neo-PAP/m, NFYC/m, NGEP, NMP22, NPM/ALK, N-Ras/m, NSE, NY-ESO-1, NY-ESOB, OA1, OFA-iLRP, OGT, OGT/m, OS-9, OS-9/m, osteocalcin, osteopontin, pi 5, p190 minor bcr-abl, p53, p53/m, PAGE-4, PAI-1, PAI-2, PAP, PART-1, PATE, PDEF, Pim-1 kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD168, RU1, RU2, S-100, SAGE, SART-1, SART-2, SART-3, SCC, SIRT2/m, Sp17, SSX-1, SSX-2/HOM-MEL-40, SSX-4, STAMP-1, STEAP-1, survivin, survivin-2B, SYT-SSX-1, SYT-SSX-2, TA-90, TAG-72, TARP, TEL-AML1, TGF-β, TGF-βRII, TGM-4, TPI/m, TRAG-3, TRG, TRP-1, TRP-2/6b, TRP/INT2, TRP-p8, tyrosinase, uPA, VEGFR1, VEGFR-2/FLK-1, WT1, and an immunoglobulin genotype of lymphoid cells or a T cell receptor genotype of lymphoid cells, or a homolog, fragment, variant, or derivative of any of the tumor antigens.

In the present invention, the tumor antigen may be selected from the group consisting of NY-ESO-1, HER-2/neu, MAGE-1, tyrosinase, MUC1, CEA, Mam-A, hTERT, Sialyl-Tn, WTl, alpha-fetoprotein, CA-125, gp-100, p53, Ras, Src, EGFRvIII, PSMA, GD2, Bcr-abl, survivin, PSA, EphA2, PAP, AFP, EpCAM, ALK, mesothelin, PSCA, MART-1, Melan-A, SCP-1, SPAG9, AKAP4, and OY-TES-1, but is not limited thereto.

In the present invention, the pathogenic antigen may be selected from the group consisting of bacterial, viral, fungal, and protozoan antigens.

The pathogenic antigen may be derived from influenza virus, respiratory syncytial virus (RSV), coronavirus, herpes simplex virus (HSV), human papillomavirus (HPV), human immunodeficiency virus (HIV), Plasmodium, Staphylococcus aureus, dengue virus, Chlamydia trachomatis, cytomegalovirus (CMV), hepatitis B virus (HBV), Mycobacterium tuberculosis, rabies virus, and yellow fever virus, or any isoforms, homologs, fragments, variants, or derivatives of proteins thereof.

In the present invention, the viral antigen may be a coronavirus antigen, but is not limited thereto.

The coronavirus may be human coronavirus 229E (HCoV-229E), human coronavirus OC43 (HCoV-OC43), severe acute respiratory syndrome coronavirus (SARS-CoV), human coronavirus NL63 (HCoV-NL63, New Haven coronavirus), human coronavirus HKU1, Middle East respiratory syndrome coronavirus (MERS-CoV), or severe acute respiratory syndrome coronavirus 2 (SARS-CoV-2).

In the present invention, the 3'-UTR may be selected from the group consisting of a β-globin 3'-UTR; a CYBA 3'-UTR; an albumin 3'-UTR; a growth hormone (GH) 3'-UTR; a VEEV 3'-UTR; a hepatitis B virus (HBV) 3'-UTR; an α-globin 3'-UTR; a DEN 3'-UTR; a PAV barley yellow dwarf virus (BYDV-PAV) 3'-UTR; an elongation factor 1 alpha 1 (EEF1A1) 3'-UTR; a manganese superoxide dismutase (MnSOD) 3'-UTR; a β-subunit of mitochondrial H(+)-ATP synthase (β-mRNA) 3'-UTR; a GLUT1 3'-UTR; a MEF2A 3'-UTR; a β-F1-ATPase 3'-UTR; a 3'-UTR represented by the nucleotide sequence of SEQ ID NO: 13; a 3'-UTR represented by the nucleotide sequence of SEQ ID NO: 14; functional fragments thereof; and combinations thereof, but is not limited thereto.
SEQ ID NO: 13: 3'UTR_EGFP 
5′-
GAUCUCGAGCUCAAGCUUCGAAUUCUGCAGUCGACGGUACCGCGGGCCCGGGAUCC
ACCGGAUCUAGAUAACUGAUCAUAAUCAGCCAUACCACAUUUGUAGAGGUUUUACU
UGCUUUAAAAAACCUCCCACACCUCCCCCUGAACCUGAAACAUAAAAUGAAUGCAA
UUGUUGUUGUUAACUUGUUUAUUGCAGCUUAUAAUGGUUACAAAUAAAGCAAUAGC
AUCACAAAUUUCACAAAUAAAGCAUUUUUUUCACUGCAUUCUAGUUGUGGUUUGUC
CAAACUCAUCAAUGUAUCUUA-3′
SEQ ID NO: 14: 3'UTR_Spike

5'-
AGGCUGGAGCCUCGGUGGCCAUGCUUCUUGCCCCUUGGGCCUCCCCCCAGCCCCUC
CUCCCCUUCCUGCACCCGUACCCCCGUGGUCUUUGAAUAAAGUCUGACUUAAG/sd
hRNA_sequence/UCUAGACAGACAUGAUAAGAUACAUUGAUGAGUUUGGACAAA
CCACAACUAGAAUGCAGUGAAAAAAAUGCUUUAUUUGUGAAAUUUGUGAUGCUAUU

GCUUUAUUUGUAACCAUUAUAAGCUGCAAUAAACAAGUU-3'

As used herein, the term "3'-UTR" typically refers to the portion of mRNA located between the protein-coding region (i.e., open reading frame, coding region) and the poly(A) sequence. The 3'-UTR of mRNA is not translated into an amino acid sequence. The 3'-UTR sequence is generally encoded by the gene that is transcribed into each mRNA during gene expression. This genomic sequence is first transcribed into an immature mRNA including optional introns. The immature mRNA is subsequently processed into mature mRNA during maturation. Such maturation includes steps such as 5'-capping, splicing of immature mRNA to remove optional introns, and 3'-end modifications including polyadenylation of the 3' end of the immature mRNA, as well as optional endonucleolytic or exonucleolytic cleavage.

In the present invention, the 3'-UTR is located downstream of the stop codon of the protein-coding region, preferably immediately downstream of the stop codon of the protein-coding region, and corresponds to the sequence of a mature mRNA extending to the 5' side of the poly(A) sequence, preferably to the nucleotide immediately adjacent to the 5' end of the poly(A) sequence. The term "corresponding" means that the 3'-UTR sequence may be an RNA sequence as in the mRNA sequence used to define the 3'-UTR, or a DNA sequence corresponding to such an RNA sequence.

In the present invention, the nucleic acid construct further includes a poly(A) tail or a poly(A) tail-like sequence. In additional embodiments, a terminal group on the poly(A) tail may be incorporated for stabilization. In other embodiments, the poly(A) tail includes a des-3' hydroxyl tail.

During RNA processing, a long chain of adenine nucleotides (poly(A) tail) may be added to a polynucleotide, such as an mRNA molecule, to increase stability. Immediately after transcription, the 3' end of the transcript may be cleaved to generate a free 3' hydroxyl group. Subsequently, poly(A) polymerase adds a chain of adenine nucleotides to the RNA. The process, referred to as polyadenylation, adds a poly(A) tail having, for example, a length of about 80 to about 250 residues (a length of about 80, 90, 100, 110, 120, 130, 140, 150, 160, 170, 180, 190, 200, 210, 220, 230, 240, or 250 residues). The poly(A) tail may also be added after the construct is exported from the nucleus.

According to the present invention, a terminal group on the poly(A) tail may be incorporated for stabilization. The polynucleotide of the present invention may include a des-3' hydroxyl tail. The polynucleotide of the present invention may further include structural moieties or 2'-O-methyl modifications as described by Junjie Li et al. (Current Biology, Vol. 15, 1501-1507, Aug. 23, 2005), the contents of which are incorporated herein by reference in their entirety.

A defined poly(A) tail length provides certain advantages to the polynucleotide of the present invention. In general, the poly(A) tail, if present, has a length of greater than 30 nucleotides. In other embodiments, the poly(A) tail has a length greater than 35 nucleotides (e.g., greater than at least about 35, 40, 45, 50, 55, 60, 70, 80, 90, 100, 120, 140, 160, 180, 200, 250, 300, 350, 400, 450, 500, 600, 700, 800, 900, 1,000, 1,100, 1,200, 1,300, 1,400, 1,500, 1,600, 1,700, 1,800, 1,900, 2,000, 2,500, and 3,000 nucleotides).

In some embodiments, the polynucleotide or a region thereof includes about 30 to about 3,000 nucleotides (e.g., 30 to 50, 30 to 100, 30 to 250, 30 to 500, 30 to 750, 30 to 1,000, 30 to 1,500, 30 to 2,000, 30 to 2,500, 50 to 100, 50 to 250, 50 to 500, 50 to 750, 50 to 1,000, 50 to 1,500, 50 to 2,000, 50 to 2,500, 50 to 3,000, 100 to 500, 100 to 750, 100 to 1,000, 100 to 1,500, 100 to 2,000, 100 to 2,500, 100 to 3,000, 500 to 750, 500 to 1,000, 500 to 1,500, 500 to 2,000, 500 to 2,500, 500 to 3,000, 1,000 to 1,500, 1,000 to 2,000, 1,000 to 2,500, 1,000 to 3,000, 1,500 to 2,000, 1,500 to 2,500, 1,500 to 3,000, 2,000 to 3,000, 2,000 to 2,500, and 2,500 to 3,000 nucleotides).

In some embodiments, the poly(A) tail is designed relative to the total length of the polynucleotide or the length of a specific region of the polynucleotide. This design may be based on the length of the coding region, specific features or regions, or the length of the ultimate product expressed from the polynucleotide.

In this regard, the poly(A) tail may be 10, 20, 30, 40, 50, 60, 70, 80, 90, or 100% longer than the polynucleotide or a feature thereof. Alternatively, the poly(A) tail may be designed as a fraction of the polynucleotide to which it belongs. In this regard, the poly(A) tail may constitute at least 10, 20, 30, 40, 50, 60, 70, 80, or 90% of the total length of the construct, a region of the construct, or the total length of the construct excluding the poly(A) tail. Furthermore, engineered binding sites and conjugation of polynucleotides to poly(A) -binding proteins may enhance expression.

Additionally, multiple distinct polynucleotides may be linked together via their 3' ends through PABP (poly(A)-binding protein), using modified nucleotides at the 3' end of the poly (A) tail. Transfection experiments may be conducted in appropriate cell lines, and protein production may be assayed by ELISA at 12, 24, 48, 72 hours, and 7 days after transfection.

In some embodiments, the polynucleotide of the present invention is designed to include a poly(A)-G quartet region. A G-quartet is a cyclic hydrogen-bonded arrangement of four guanine nucleotides that may be formed by G-rich sequences in both DNA and RNA. In this context, the G-quartet is incorporated at the end of the poly(A) tail. Metrics for evaluation of the resulting polynucleotide include stability, protein production, and half-life at various time points. It has been found that a poly(A)-G quartet yields protein production from mRNA at least equivalent to about 75% of that achievable using a poly(A) tail of 120 nucleotides alone.

In the present invention, the poly(A) tail-like sequence may be any nucleic acid sequence capable of performing the function of a poly(A) tail without limitation, and preferably is characterized in that at least one nucleotide other than adenine, selected from the group consisting of uracil (U), cytosine (C), and guanine (G), is inserted between multiple adenines or at the end of the poly(A) tail, but is not limited thereto.

In the present invention, the nucleic acid construct may be RNA.

In the present invention, the RNA may be selected from the group consisting of mRNA, viral RNA, self-amplifying RNA, and replicon RNA, but is not limited thereto.

In the present invention, the nucleic acid construct may include at least one backbone-modified, sugar-modified, or base-modified nucleic acid, but is not limited thereto.

### Sugar modifications:

Modified nucleosides and nucleotides that may be incorporated into modified mRNA compounds including the mRNA sequence as described herein may be modified at the sugar moiety. For example, the 2'-hydroxyl group (OH) may be modified or replaced with various "oxy" or "deoxy" substituents. Examples of "oxy"-2' hydroxyl group modifications include, but are not limited to, alkoxy or allyloxy (-OR, where R = H, alkyl, cycloalkyl, aryl, aralkyl, heteroaryl, or sugar); polyethylene glycol (PEG), -O(CH2CH2O)nCH2CH2OR; "locked" nucleic acids (LNA), in which the 2'-hydroxyl is linked to the 4' carbon of the same ribose sugar, for example via a methylene bridge; and amino groups (-O-amino, wherein the amino group, for example NRR, may be alkylamino, dialkylamino, heterocyclyl, arylamino, diarylamino, heteroarylamino, or diheteroarylamino, ethylenediamine, or polyamino) or aminoalkoxy groups.

"Deoxy" modifications may include hydrogen or amino groups (e.g., NH2; alkylamino, dialkylamino, heterocyclyl, arylamino, diarylamino, heteroarylamino, diheteroarylamino, or amino acids); or the amino group may be attached to the sugar via a linker, in which the linker includes at least one of C, N, or O atoms.

The sugar moiety may also contain one or more carbons having a stereochemical configuration opposite to that of the corresponding carbon in ribose. Thus, modified mRNA may include nucleotides containing, for example, arabinose as the sugar.

### Backbone modifications:

The phosphate backbone may be further modified in modified nucleosides and nucleotides that may be incorporated into modified mRNA compounds including the mRNA sequence as described herein. The phosphate group of the backbone may be modified by replacing one or more oxygen atoms with other substituents. The modified nucleosides and nucleotides may also include complete replacement of the unmodified phosphate moiety with modified phosphate groups as described herein. Examples of the modified phosphate group include, but are not limited to, phosphorothioate, phosphoroselenoate, boranophosphate, boranophosphate ester, hydrogen phosphonate, phosphoramidate, alkyl or aryl phosphonate, and phosphotriester. Phosphorodithioates have both non-bridging oxygen atoms replaced by sulfur.

The phosphate linker may also be modified by substitution of the bridging oxygen with nitrogen (bridged phosphoramidate), sulfur (bridged phosphorothioate), or carbon (bridged methylene phosphonate) .

### Base modifications:

Modified nucleosides and nucleotides that may be incorporated into modified mRNA compounds including the mRNA sequence as described herein may be further modified at the nucleobase moiety. Examples of nucleobases found in mRNA include, but are not limited to, adenine, guanine, cytosine, and uracil. For example, the nucleosides and nucleotides described herein may be chemically modified at the major groove face. In some embodiments, such major groove chemical modifications may include amino, thiol, alkyl, or halo groups.

In a particularly preferred embodiment of the present invention, the nucleotide analogs/modifications are preferably selected from among base-modified nucleotides including 2-amino-6-chloropurine riboside-5'-triphosphate, 2-aminopurine riboside-5'-triphosphate, 2-aminoadenosine-5'-triphosphate, 2'-amino-2'-deoxycytidine triphosphate, 2-thiocytidine-5'-triphosphate, 2-thiouridine-5'-triphosphate, 2'-fluorothymidine-5'-triphosphate, 2'-O-methylinosine-5'-triphosphate, 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-bromo-2'-deoxycytidine-5'-triphosphate, 5-bromo-2'-deoxyuridine-5'-triphosphate, 5-iodocytidine-5'-triphosphate, 5-iodo-2'-deoxycytidine-5'-triphosphate, 5-iodouridine-5'-triphosphate, 5-iodo-2'-deoxyuridine-5'-triphosphate, 5-methylcytidine-5'-triphosphate, 5-methyluridine-5'-triphosphate, 5-propynyl-2'-deoxycytidine-5'-triphosphate, 5-propynyl-2'-deoxyuridine-5'-triphosphate, 6-azacytidine-5'-triphosphate, 6-azauridine-5'-triphosphate, 6-chloropurine riboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, puromycin-5'-triphosphate, and xanthosine-5'-triphosphate.

Particularly preferred are nucleotides having base modifications selected from the group consisting of base-modified nucleotides including 5-methylcytidine-5'-triphosphate, 7-deazaguanosine-5'-triphosphate, 5-bromocytidine-5'-triphosphate, and pseudouridine-5'-triphosphate.

In some embodiments, the modified nucleosides include pyridin-4-one [0356] ribonucleoside, 5-azauridine, 2-thio-5-azauridine, 2-thiouridine, 4-thiopseudouridine, 2-thiopseudouridine, 5-hydroxyuridine, 3-methyluridine, 5-carboxymethyluridine, 1-carboxymethylpseudouridine, 5-propynyluridine, 1-propynylpseudouridine, 5-taurinomethyluridine, 1-taurinomethylpseudouridine, 5-taurinomethyl-2-thiouridine, 1-taurinomethyl-4-thiouridine, 5-methyluridine, 1-methylpseudouridine, 4-thio-1-methylpseudouridine, 2-thio-1-methylpseudouridine, 1-methyl-1-deazapseudouridine, 2-thio-1-methyl-1-deazapseudouridine, dihydrouridine, dihydropseudouridine, 2-thio-dihydrouridine, 2-thio-dihydropseudouridine, 2-methoxyuridine, 2-methoxy-4-thiouridine, 4-methoxypseudouridine, and 4-methoxy-2-thiopseudouridine.

In some embodiments, the modified nucleosides include 5-azacytidine, pseudoisocytidine, 3-methylcytidine, N4-acetylcytidine, 5-formylcytidine, N4-methylcytidine, 5-hydroxymethylcytidine, 1-methylpseudoisocytidine, pyrrolo-cytidine, pyrrolo-pseudoisocytidine, 2-thiocytidine, 2-thio-5-methylcytidine, 4-thio-pseudoisocytidine, 4-thio-1-methylpseudoisocytidine, 4-thio-1-methyl-1-deazapseudoisocytidine, 1-methyl-1-deazapseudoisocytidine, zebularine, 5-azazebularine, 5-methylzebularine, 5-aza-2-thiozebularine, 2-thiozebularine, 2-methoxycytidine, 2-methoxy-5-methylcytidine, 4-methoxypseudoisocytidine, and 4-methoxy-1-methylpseudoisocytidine.

In other embodiments, the modified nucleosides include 2-aminopurine, 2,6-diaminopurine, 7-deazaadenine, 7-deaza-8-azaadenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonylcarbamoyladenosine, N6,N6-dimethyladenosine, 7-methyladenine, 2-methylthioadenine, and 2-methoxyadenine.

In other embodiments, the modified nucleosides include inosine, 1-methylinosine, wyosine, wybutosine, 7-deazaguanosine, 7-deaza-8-azaguanosine, 6-thioguanosine, 6-thio-7-deazaguanosine, 6-thio-7-deaza-8-azaguanosine, 7-methylguanosine, 6-thio-7-methylguanosine, 7-methylinosine, 6-methoxyguanosine, 1-methylguanosine, N2-methylguanosine, N2,N2-dimethylguanosine, 8-oxoguanosine, 7-methyl-8-oxoguanosine, 1-methyl-6-thioguanosine, N2-methyl-6-thioguanosine, and N2,N2-dimethyl-6-thioguanosine.

In some embodiments, the nucleotide may be modified at the major groove face and may include substitution of hydrogen at the C-5 position of uracil with a methyl group or a halogen group. In certain embodiments, the modified nucleoside is 5'-O-(1-thiophosphate)-adenosine, 5'-O-(1-thiophosphate)-cytidine, 5'-O-(1-thiophosphate)-guanosine, 5'-O-(1-thiophosphate)-uridine, or 5'-O-(1-thiophosphate)-pseudouridine.

In further specific embodiments, the modified mRNA may include nucleoside modifications selected from the group consisting of 6-azacytidine, 2-thiocytidine, α-thiocytidine, pseudoisocytidine, 5-aminoallyluridine, 5-iodouridine, N1-methylpseudouridine, 5,6-dihydrouridine, α-thiouridine, 4-thiouridine, 6-azauridine, 5-hydroxyuridine, deoxythymidine, 5-methyluridine, pyrrolo-cytidine, inosine, α-thioguanosine, 6-methylguanosine, 5-methylcytidine, 8-oxoguanosine, 7-deazaguanosine, N1-methyladenosine, 2-amino-6-chloropurine, N6-methyl-2-aminopurine, pseudoisocytidine, 6-chloropurine, N6-methyladenosine, α-thioadenosine, 8-azidoadenosine, and 7-deazaadenosine.

In some embodiments, the base-modified nucleic acid may be selected from the group consisting of pseudouridine (Ψ), N1-methylpseudouridine (m1Ψ), 5-methyluridine (m5U), 2-thiouridine (s2U), 2'-O-methyluridine (Um), 5-methylcytidine (m5C), and 5-methoxyuridine (5moU), but is not limited thereto.

Another aspect of the present invention relates to a vaccine composition including the nucleic acid construct.

As used herein, the term "vaccine" is typically understood as a prophylactic or therapeutic substance providing at least one antigen, preferably an antigenic peptide or protein. "Providing at least one antigen" means, for example, that the vaccine includes the antigen or includes a molecule encoding the antigen. Accordingly, the vaccine of the present invention is particularly envisaged to include at least one synthetic nucleic acid (RNA) molecule encoding at least one antigenic (poly)peptide or protein as defined herein, which may be derived, for example, from a tumor antigen, bacterial, viral, fungal, or protozoan antigen, an autoantigen, an allergen, or an alloantigen, and which preferably induces an immune response to each antigen upon expression and presentation to the immune system. However, synthetic nucleic acid (RNA) molecules encoding non-antigenic (poly)peptides or proteins of interest may also be used in the vaccine of the present invention.

In the present invention, the nucleic acid construct may be complexed with one or more lipids to form lipid nanoparticles or liposomes.

In the present invention, the synthetic nucleic acid (RNA) molecule may be provided in a complexed or associated form with one or more (poly)cationic compounds, preferably (poly)cationic polymers, (poly)cationic peptides or proteins (e.g., protamine), (poly)cationic polysaccharides, and/or (poly)cationic lipids. In this context, the terms "complexed" or "associated" mean that at least one synthetic nucleic acid (RNA) molecule forms a substantially stable combination with the one or more compounds as part of a larger complex or assembly without covalent bonding.

### Lipids

According to preferred embodiments, the synthetic nucleic acid (RNA) molecule of the present invention is complexed or associated with lipids (particularly cationic and/or neutral lipids) to form one or more lipid nanoparticles or liposomes. Accordingly, in some embodiments, the synthetic nucleic acid (RNA) molecule of the present invention may be provided in the form of a lipid-based formulation, particularly liposomes and/or lipid nanoparticles including the synthetic nucleic acid (RNA) molecule.

### Lipid nanoparticles (LNPs)

In some preferred embodiments, the synthetic nucleic acid (RNA) molecule of the present invention is complexed or associated with lipids (particularly cationic and/or neutral lipids) to form one or more lipid nanoparticles.

Preferably, the lipid nanoparticles (LNPs) include: (a) at least one nucleic acid construct (RNA) of the present invention, (b) a cationic lipid, (c) an aggregation-reducing agent (e.g., polyethylene glycol (PEG)-lipid or PEG-modified lipid), (d) optionally a non-cationic lipid (e.g., neutral lipid), and (e) optionally a sterol.

In some embodiments, the LNPs may include, in addition to at least one nucleic acid construct (RNA) of the present invention: (i) at least one cationic lipid; (ii) a neutral lipid; (iii) a sterol (e.g., cholesterol); and (iv) a PEG-lipid, at a molar ratio of about 20-60% cationic lipid:5-25% neutral lipid:25-55% sterol:0.5-15% PEG-lipid.

In some embodiments, the nucleic acid construct (RNA) of the present invention may be formulated with an aminoalcohol lipidoid. The aminoalcohol lipidoid that may be used in the present invention may be prepared by methods described in U.S. Patent No. 8,450,298, the entire contents of which are incorporated herein by reference.

### Liposomes

In some embodiments, the synthetic nucleic acid (RNA) molecule of the present invention is formulated as a liposome. Cationic lipid-based liposomes may form complexes with negatively charged nucleic acids (e.g., RNA) through electrostatic interactions, thereby generating complexes with biocompatibility, low toxicity, and scalability for *in vivo* clinical applications. Liposomes may fuse with the plasma membrane for uptake; once inside the cell, liposomes are processed via phagocytic pathways and the nucleic acid is subsequently released into the cytoplasm from endosomes/vesicles. Liposomes are essentially analogs of biological membranes and may be prepared from both natural and synthetic phospholipids, which has led to their longstanding recognition as excellent drug delivery vehicles due to superior biocompatibility.

Liposomes typically consist of a lipid bilayer surrounding an aqueous core, wherein the bilayer may include cationic, anionic, or neutral (phospho)lipids and cholesterol. Both the lipid bilayer and the aqueous space may encapsulate hydrophobic or hydrophilic compounds. Liposomes may have one or more lipid bilayers. Liposomes may be unilamellar, having a single layer, or multilamellar, having multiple layers.

*In vivo* liposome properties and behavior may be modified by adding hydrophilic polymer coatings, such as polyethylene glycol (PEG), to the liposome surface to provide steric stabilization. Additionally, liposomes may be used for targeted delivery by attaching ligands (e.g., antibodies, peptides, carbohydrates) to the surface thereof or to the ends of attached PEG chains.

Liposomes are typically spherical vesicles with sizes ranging from about 20 nm to several micrometers. Liposomes may include multilamellar vesicles (MLVs) with diameters of several hundred nanometers, including concentric bilayers separated by narrow aqueous compartments, small unicellular vesicles (SUVs) with diameters less than 50 nm, and large unilamellar vesicles (LUVs) with diameters between about 50 and 500 nm, although not limited thereto. Liposome design may include, but is not limited to, opsonins or ligands to enhance attachment of liposomes to diseased tissues or to trigger events such as endocytosis. Liposomes may also be formulated at low or high pH to enhance delivery of pharmaceutical formulations.

In the present invention, the vaccine composition may further include at least one adjuvant or active agent.

In the broadest sense, an "adjuvant" or "adjuvant component" is typically a pharmacological and/or immunological agent capable of modifying, for example enhancing, the effect of another active agent such as a therapeutic or vaccine. In this context, an "adjuvant" may be understood as any compound capable of facilitating administration and delivery of the vaccine composition of the present invention. Specifically, an adjuvant preferably enhances the immunostimulatory properties of the administered vaccine. Furthermore, such adjuvants, without being bound thereto, may initiate or increase an immune response of the innate immune system, namely a non-specific immune response.

An "adjuvant" does not typically induce an adaptive immune response. To date, an "adjuvant" has not been considered to qualify as an antigen. Upon administration, the vaccine of the present invention typically initiates an adaptive immune response due to the antigenic peptide or protein encoded by at least one coding sequence of the synthetic nucleic acid (RNA) molecule contained in the vaccine.

Suitable adjuvants are known to those skilled in the art and may be selected from among adjuvants suitable for the present case, namely adjuvants that facilitate the induction of an immune response in mammals, and may include, but are not limited to, TDM, MDP, muramyl dipeptide, Pluronic, alum solution, aluminum hydroxide, ADJUMER^{™} (polyphosphazene); aluminum phosphate gel; glucans from algae; algammulin; aluminum hydroxide gel (alum); high protein-adsorbing aluminum hydroxide gel; low-viscosity aluminum hydroxide gel; AF or SPT (an emulsion of squalane (5%), Tween 80 (0.2%), Pluronic L121 (1.25%), phosphate-buffered saline, pH 7.4); or AVRIDINE^{™} (propanediamine).

Still another aspect of the present invention relates to the use of the vaccine composition for the prevention of a disease.

As used herein, "disease" refers to an abnormal condition arising from tumor antigens, bacterial, viral, fungal, or protozoan antigens, autoantigens, allergens, or alloantigens, and may include, but is not limited to, cancer, tumors, autoimmune diseases, inflammatory diseases, viral infections, bacterial infections, fungal infections, and protozoan infections.

As used herein, "prevention" refers to any act of inhibiting the onset of a disease or delaying progression thereof by administration of the vaccine composition of the present invention.

Yet another aspect of the present invention relates to a method of preventing a disease, including administering the vaccine composition.

Still yet another aspect of the present invention relates to the use of the vaccine composition for the manufacture of a medicament for the prevention of a disease.

A better understanding of the present invention may be obtained through the following examples. There examples are merely set forth to illustrate the present invention, and are not to be construed as limiting the scope of the present invention, as will be apparent to those skilled in the art.

The experimental methods commonly used in the following Examples are specifically described below.

### Experiment 1: PCR for IVT template DNA preparation

PCR is performed using a plasmid containing an EGFP or Spike ORF sequence and a primer including a T7 promoter sequence, with KAPA HiFi HotStart ReadyMix (Roche, KK2601), to obtain template DNA for *in vitro* transcription (IVT). After PCR, the PCR template is confirmed on a 1-1.5% agarose gel, and the IVT T7 template DNA is purified using a QIAquick PCR Purification Kit (Qiagen, 28104).

The IVT PCR template sequences for synthesis of EGFP or Spike mRNA are as follows.
SEQ ID NO: 15: EGFP_IVT template 
EGFP: 5′-
TAATACGACTCACTATAGGGTTTAGTGAACCGTCAGATCCGCTAGCGCTAC
CGGTCGCCACCATGGTGAGCAAGGGCGAGGAGCTGTTCACCGGGGTGGTGCCCATC
CTGGTCGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACCGGCA
AGCTGCCCGTGCCCTGGCCCACCCTCGTGACCACCCTGACCTACGGCGTGCAGTGC
TTCAGCCGCTACCCCGACCACATGAAGCAGCACGACTTCTTCAAGTCCGCCATGCC
 
CGAAGGCTACGTCCAGGAGCGCACCATCTTCTTCAAGGACGACGGCAACTACAAGA
CCCGCGCCGAGGTGAAGTTCGAGGGCGACACCCTGGTGAACCGCATCGAGCTGAAG
GGCATCGACTTCAAGGAGGACGGCAACATCCTGGGGCACAAGCTGGAGTACAACTA
CAACAGCCACAACGTCTATATCATGGCCGACAAGCAGAAGAACGGCATCAAGGTGA
ACTTCAAGATCCGCCACAACATCGAGGACGGCAGCGTGCAGCTCGCCGACCACTAC
CAGCAGAACACCCCCATCGGCGACGGCCCCGTGCTGCTGCCCGACAACCACTACCT
GAGCACCCAGTCCGCCCTGAGCAAAGACCCCAACGAGAAGCGCGATCACATGGTCC
TGCTGGAGTTCGTGACCGCCGCCGGGATCACTCTCGGCATGGACGAGCTGTACAAG
TCCGGATAA/sdhRNA_seqeunce/AGATCTCGAGCTCAAGCTTCGAATTCTGCAG
TCGACGGTACCGCGGGCCCGGGATCCACCGGATCTAGATAACTGATCATAATCAGCC
ATACCACATTTGTAGAGGTTTTACTTGCTTTAAAAAACCTCCCACACCTCCCCCTGA
ACCTGAAACATAAAATGAATGCAATTGTTGTTGTTAACTTGTTTATTGCAGCTTATA
ATGGTTACAAATAAAGCAATAGCATCACAAATTTCACAAATAAAGCATTTTTTTCAC
TGCATTCTAGTTGTGGTTTGTCCAAACTCATCAATGTATCTTA-3′
SEQ ID NO: 16: Spike_IVT_Template

Spike: 5'-
TAATACGACTCACTATAAGGAGAGGGAAATAAGAGAGAAAAGAAGAGTAAG
AAGAAATATAAGAGCCACCATGTTCGTGTTCCTGGTGCTGCTGCCTCTGGTGAGCA
GCCAGTGCGTGAATCTGATTACAAGAACTCAGGAGTACACTAACTCTTTCACCAGA
GGAGTGTACTACCCCGATAAGGTGTTTAGGTCAAGCGTGCTGCACATGACTCAGGA
CCTGTTCCTGCCTTTCTTTTCTAATGTGACCTGGTTCCACGCAATCCACGTGAGCG
GCAGCAACGGCACGAAATATTTCGATAATCCTGTGCTGCCCTTCAACGACGGCGTG
TACTTCGCCTCCACAGAGAAGTCAAACATTATTCGGGGGTGGATCTTCGGCACCAC
ACTGGACAGTAAAACCCAGTCCCTGCTGATCGTGAACAATGCTACCAACGTGGTGA

TTAAGGTGTGCTACTTTCAGTTCTGCAATGATCCATTCCTGGATGTGTACTACCAC

AAGAACAATAAGTCCTGGATGGAAAGCGAATTCAGAGTGTATTCCTCTGCAAATAA

CTGTACATTCGAGTACGTGTCCAAGCCATTCCTCATGGACCTTGAAGGCAAGCAGG

GGAATTTTAAAAACCTGCGGGAGTTTGTGTTTAAGAATATCGATGGCTACTTCAAG

ATTTATTCCAAACACACTCCCATCAACCTGACCAGGGACCTGCCCCAGGGCTTTTC

CGCCCTGGAGCCCCTGGTGGACCTGCCCATTGGCATTAACATTACAAGATTCCAGA

CCCTGCTGGCTCTGCACAGGTCCTATCTGACACCTGGCGATAGCAGCAATGGCTGG

ACCGTCGGGGCAGCCGCCTATTACGTGGGATACCTGCAGCCCCGGACCTTTCTGCT

GAAGTACAATGAGAACGGCACAATCACAGACGCTGTGGACTGCGCCCTGGACCCAC

TGAGCGAGACTAAGTGCACTCTGAAATCATTCACTGTGGAGAAGGGGATTTATCAG

ACATCTAACTTCAGAGTCCAGCCTACCGAGAGTATCGTGAGGTTCCCCAACATCAC

CAACCTGTGCCCTTTTCACGAGGTGTTTAACGCCACCACCTTCGCCTCAGTGTATG

CCTGGAACAGGAAGAGAATCAGCAATTGCGTGGCAGACTACTCCGTGCTGTACAAC

TTCGCACCCTTCTTCGCCTTTAAGTGTTACGGCGTGAGCCCCACCAAACTGAACGA

TCTGTGCTTCACAAATGTGTACGCCGATAGTTTTGTGATCCGGGGGAATGAGGTGT

CTCAAATCGCCCCCGGCCAGACCGGAAACATCGCTGATTATAACTACAAGCTGCCC

GATGATTTTACCGGGTGCGTGATCGCCTGGAATAGTAATAAGCTGGATAGCAAAGT

GTCTGGCAACTATAACTATCTGTACCGGCTGTTCAGGAAATCCAAGCTGAAGCCCT

TCGAGCGCGACATCTCTACCGAAATTTACCAGGCCGGTAACAAGCCATGTAACGGC

GTGGCTGGCTTTAATTGCTACTTCCCACTGCAGAGCTACGGGTTCCGGCCCACCTA

TGGGGTGGGGCATCAGCCCTACCGCGTGGTGGTGCTGTCCTTCGAGCTGCTGCACG

CCCCAGCTACCGTGTGCGGGCCCAAGAAGTCAACTAATCTGGTCAAGAACAAGTGT

GTCAACTTCAACTTCAATGGCCTGACTGGGACAGGCGTGCTCACCGAGAGCAACAA

GAAATTCCTGCCTTTTCAGCAGTTCGGCAGGGACATCGCCGACACCACAGATGCCG

TTCGGGACCCACAGACCCTGGAAATCCTGGATATTACTCCATGCAGCTTCGGCGGC

GTGTCCGTGATTACACCTGGAACCAACACCAGCAACCAGGTGGCCGTGCTGTACCA
GGGCGTGAACTGCACCGAGGTGCCTGTGGCAATACACGCCGACCAGCTGACTCCCA
CATGGCGAGTGTATTCAACCGGCTCCAATGTGTTTCAGACTAGAGCCGGCTGCCTG
ATCGGAGCTGAGCACGTGAATAACTCTTATGAGTGCGATATCCCCATCGGAGCCGG
CATCTGCGCATCCTATCAGACACAGACAAACAGCCACGCCAGCGTGGCCTCTCAAA
GCATCATTGCCTACACAATGAGCCTGGGCGCCGAGAACTCCGTTGCTTATAGTAAT
AATAGCATTGCAATCCCTACCAACTTCACCATCAGCGTGACCACAGAGATTCTGCC
TGTGAGCATGACCAAGACCTCCGTGGACTGTACCATGTACATCTGTGGCGACTCGA
CTGAATGCAGCAACCTGCTGCTCCAATACGGCAGCTTCTGCACACAGCTGAACCGG
GCTCTGACCGGCATCGCCGTGGAACAGGACAAGAACACCCAAGAGGTGTTTGCCCA
GGTGAAGCAGATTTACAAGACCCCTCCCATCAAGGACTTCGGCGGTTTCAACTTTA
GCCAGATCCTGCCTGATCCTTCTAAACCCAGCAAGCGGAGCCCCATCGAGGACCTG
CTGTTCAACAAGGTGACCCTGGCTGACGCCGGATTCATCAAGCAGTATGGCGACTG
TCTGGGCGATATCGCTGCCAGAGACCTCATCTGTGCCCAGAAGTTCAACGGACTGA
CTGTGCTACCACCCTTACTGACCGATGAGATGATCGCTCAGTACACATCTGCCCTG
CTGGCTGGGACCATCACCAGCGGCTGGACATTCGGCGCCGGCCCCGCCCTGCAAAT
CCCGTTTCCTATGCAGATGGCCTATAGATTCAACGGAATCGGCGTGACCCAGAACG
TCCTGTATGAGAACCAGAAGCTGATCGCTAATCAGTTCAACAGCGCAATCGGCAAA
ATCCAAGACTCTCTGAGCAGCACACCCTCCGCTCTGGGCAAGCTGCAGGATGTGGT
GAACCAAAACGCCCAAGCCCTTAACACCCTGGTGAAACAGCTGAGCAGCAACTTTG
GTGCCATCAGCAGCGTGCTTAACGACATTCTGTCTAGGCTGGACCCCCCTGAGGCC
GAAGTGCAGATCGACAGACTGATCACCGGAAGACTGCAGAGCCTGCAAACCTACGT
GACCCAACAGTTGATCAGAGCCGCTGAGATCCGGGCGAGCGCCAACCTGGCCGCCA
CAAAGATGAGCGAATGTGTGCTGGGGCAGAGCAAAAGAGTGGACTTCTGCGGCAAA
GGCTATCACCTGATGAGCTTTCCTCAGAGCGCCCCCCACGGCGTGGTGTTCCTGCA

CGTGACATACGTGCCTGCTCAGGAGAAGAACTTCACCACCGCCCCTGCCATCTGCC

ACGACGGCAAGGCTCACTTCCCAAGAGAAGGCGTGTTCGTCAGCAATGGCACCCAC

TGGTTCGTGACCCAAAGAAACTTCTACGAGCCTCAGATCATCACAACCGACAACAC

ATTCGTGAGCGGGAACTGCGACGTGGTAATCGGCATTGTGAACAATACCGTGTACG

ACCCTCTGCAGCCAGAACTGGATTCCTTCAAGGAAGAGCTGGACAAGTACTTTAAG

AACCACACAAGCCCTGACGTGGACCTGGGCGACATAAGCGGTATCAACGCCTCAGT

GGTCAACATCCAGAAAGAGATCGACAGGTTAAACGAAGTGGCCAAGAACCTGAATG

AGTCCCTGATCGATCTGCAGGAGCTGGGCAAGTACGAACAGTACATCAAGTGGCCT

TGGTACATCTGGCTGGGATTCATTGCCGGATTGATCGCCATCGTCATGGTGACCAT

CATGCTGTGCTGCATGACGAGCTGCTGCTCTTGCCTGAAGGGCTGCTGTAGCTGTG

GCTCCTGTTGTAAATTCGACGAAGATGACAGCGAGCCCGTGCTGAAGGGAGTGAAG

CTGCATTACACCTGATAATAGGCTGGAGCCTCGGTGGCCATGCTTCTTGCCCCTTG

GGCCTCCCCCCAGCCCCTCCTCCCCTTCCTGCACCCGTACCCCCGTGGTCTTTGAA

TAAAGTCTGACTTAAG/sdhRNA_sequence/TCTAGACAGACATGATAAGATAC

ATTGATGAGTTTGGACAAACCACAACTAGAATGCAGTGAAAAAAATGCTTTATTTG

TGAAATTTGTGATGCTATTGCTTTATTTGTAACCATTATAAGCTGCAATAAACAAG

TT-3′

### Experiment 2: In vitro transcription

*In vitro* transcription (IVT) is a process for synthesizing mRNA or sdhRNA.

For mRNA synthesis, the prepared template DNA is reacted with T7 RNA polymerase, buffer, NTPs (including natural and chemically modified forms), and other IVT components under conditions provided by the manufacturer at 37°C for 30 minutes (using a HiScribe^{™} T7 ARCA mRNA Kit (with tailing), NEB E2060S). After completion of reaction, DNase I is added at 1 U per 1 µg of DNA and incubated at 37°C for 15 minutes to remove the template DNA. Subsequently, poly(A) tailing is additionally performed at 37°C for 30 minutes on the synthesized mRNA using *E. coli* poly(A) polymerase.

For sdhRNA synthesis, the prepared template DNA is reacted with T7 RNA polymerase, buffer, NTPs (including natural and chemically modified forms), and other IVT components under conditions provided by the manufacturer at 37°C for 6 hours (using a MEGAscript^{™} T7 Transcription Kit, Invitrogen^{™} AM1334). After completion of reaction, DNase I is added at 1 U per 1 µg of DNA and incubated at 37°C for 15 minutes to remove the template DNA.

The resulting IVT products are purified using a Monarch RNA Cleanup Kit (NEB, 2040L) according to the manufacturer's instructions. The purified mRNA or sdhRNA is quantified using a NanoDrop spectrophotometer or UV/Vis absorbance, and the band pattern is confirmed by agarose gel or urea-PAGE analysis.

### Experiment 3: Cloning of sdhRNA into 3'-UTR

To incorporate the sdhRNA sequence into the 3'-UTR of EGFP or Spike mRNA, a strategy is employed in which a plasmid containing the EGFP or Spike sequence is digested using a restriction enzyme, followed by ligation with an sdhRNA containing a cloning site. For EGFP mRNA, the plasmid is digested using BspEI and BglII, and for Spike mRNA, the plasmid is digested using AflII and XbaI, followed by purification of the digested plasmid using a QIAquick Gel Extraction Kit (Qiagen 28704). The purified plasmid is ligated with sdhRNA containing the cloning site using T4 DNA ligase (NEB, M0202S). Thereafter, transformation into a DH5α strain is performed, colonies are picked, and plasmids are extracted and subjected to Sanger sequencing to verify proper cloning of the sdhRNA. Thereafter, using the plasmid incorporating the sdhRNA, template PCR (Experiment 1) and IVT (Experiment 2) are performed in the same manner to synthesize sdhRNA-incorporating mRNA.

### Experiment 4: Western blotting

Cells transfected with mRNA and sdhRNA are collected and lysed by sonication, followed by protein quantification via a BCA assay using a Pierce^{™} BCA Protein Assay Reagent (Thermo Fisher, 23223). After gel electrophoresis using a 10% SDS-PAGE gel, Amersham transfer is performed. Thereafter, primary and secondary antibody reactions are carried out for the target proteins, and finally, protein expression levels are measured using a Pierce^{™} ECL Western Blotting Substrate. The primary antibodies for the proteins measured in the present development are as follows: PKR (Cell Signaling Technology, 12297), pPKR (Abcam, 81303), eIF2α (Cell Signaling Technology, 5324), p-eIF2α (Cell Signaling Technology, 9721), TUBB (Cell Signaling Technology, 86298), PARP & cleaved PARP (Cell Signaling Technology, 9542), GFP (Sigma Aldrich, G1544), and Spike protein (Genetex, GTX632604).

### Experiment 5: RNA extraction and RT-qPCR

Cells transfected with mRNA and sdhRNA are treated with TRIzol (Ambion 15596018) and subjected to IPA precipitation, followed by treatment with DNase I (Takara 2270) to remove residual DNA in the cells. Thereafter, RNA is dissolved in TDW after EtOH precipitation, washing, and drying. cDNA is synthesized using the extracted RNA, Reverse Transcriptase (Thermo Fisher EP0442), buffer, and NTPs. Subsequently, gene expression levels are compared via real-time quantitative PCR using a SensiFAST SYBR Lo-Rox Kit (Bioline BIO-94020).

### Example 1: Evaluation of importance of PKR regulation in protein expression induced by exogenous mRNA

**The present** invention proposed the introduction of sdhRNA to regulate activation of PKR, an innate immune protein, as an mRNA therapeutic/vaccine RNA construct platform technology. To demonstrate the importance of PKR in the expression of an antigen encoded by exogenous mRNA and in innate immune activation induced by exogenous RNA, a PKR knockout (KO) cell line, in which PKR was deleted, was constructed and utilized using a CRISPR-Cas9 system.

EGFP mRNA (Experiment 2) was administered to A549 lung cancer cells (ATCC) and PKR KO A549 cells using a Lipofectamine 3000 transfection reagent (Invitrogen^{™} L3000015), and the expression pattern of the EGFP antigen and cell death were then analyzed.

Therefore, it was first confirmed by Western blotting (Experiment 4) that PKR expression was completely abolished in PKR KO cells. Upon administration of EGFP mRNA, PKR activation was observed only in A549 wild-type (WT) cells, and phosphorylation of eIF2α (p-eIF2α), a downstream effector of PKR activation, was not observed in PKR KO cells (FIG. 2A).

In addition, fluorescence microscopy and Western blotting (Experiment 4) confirmed that expression of the EGFP protein encoded by the EGFP mRNA was increased in PKR KO cells (FIG. 2B). Furthermore, based on results of evaluation of cytotoxicity of mRNA administration using a CCK-8 assay kit (Dojindo CK04) according to the manufacturer's instructions, significantly higher cell viability was observed in PKR KO cells, confirming the importance of PKR inhibition in mRNA-based therapeutic technologies (FIG. 2C).

### Example 2: Evaluation of antigen expression enhancement and modulation of innate immunity by EGFP mRNA incorporating nc886

An nc886 sequence (SEQ ID NO: 9), known to inhibit the activation of the innate immune protein PKR, was incorporated into the 3'-UTR of an EGFP nucleic acid construct, and a PCR product with a T7 promoter attached thereto was constructed using KAPA HiFi HotStart ReadyMix (Roche KK2601) to enable IVT (Experiments 1 and 3). To evaluate whether nc886-mediated modulation of innate immune activation is effective in the immune response to exogenous mRNA, EGFP mRNA having the nc886 sequence incorporated into the 3'-UTR was synthesized using a HiScribe^{™} T7 ARCA mRNA Kit (with tailing) (NEB E2060S) (Experiment 2). The EGFP mRNA was administered to A549 cells at a concentration of 400 ng/ml using a Lipofectamine 3000 transfection reagent (Invitrogen^{™} L3000015), and after 24 hours, expression of the EGFP protein and the innate immune response were analyzed by fluorescence microscopy, Western blotting (Experiment 4), and RT-qPCR (Experiment 5).

Therefore, comparison of experiments in which mRNA having nc886 incorporated into the 3'-UTR and nc886 RNA were each co-synthesized with EGFP mRNA and separately administered to cells showed that coadministration of nc886 RNA and EGFP mRNA significantly increased antigen (EGFP) expression (FIGs. 3A and 3B) and suppressed pPKR (FIG. 3C).

### Example 3: Evaluation of antigen expression enhancement and modulation of innate immunity by EGFP mRNA incorporating multiple nc886 sequences

No significant improvement in antigen expression or suppression of innate immune responses was observed in EGFP mRNA incorporating a single nc886 sequence. Accordingly, to evaluate whether an improved effect could be achieved by incorporating multiple nc886 sequences, three nc886 template DNAs were ligated and, through cloning (Experiment 3), an EGFP nucleic acid construct (EGFP-nc886X3) was designed in which three nc886 sequences were repetitively incorporated into the 3' UTR, and incorporation of the three nc886 sequences was confirmed by Sanger sequencing and 1% agarose gel (FIG. 4A). Thereafter, EGFP-nc886 mRNA and EGFP-nc886X3 mRNA were each synthesized via IVT (Experiment 2), and then administered to A549 cells using a Lipofectamine 3000 transfection reagent (Invitrogen^{™} L3000015). Subsequently, EGFP protein expression and innate immune responses were compared through fluorescence microscopy, Western blotting (Experiment 4), and RT-qPCR (Experiment 5).

Therefore, it was confirmed that, upon administration of EGFP-nc886X3 mRNA, PKR activation and downstream phosphorylation of eIF2α were most significantly reduced (FIGs. 4B to 4D).

### Example 4: Development of immunoregulatory sdhRNA

sdhRNAs were synthesized using an sdhRNA DNA library including 1,840 diverse hairpin structures capable of binding to PKR (Kim K et al., A quantitative map of human primary microRNA processing sites, Mol. Cell, 2021, 34320405) as template DNA via IVT (Experiment 2), and the binding affinity between the sdhRNAs and PKR was analyzed.

For this purpose, FLAG-PKR was expressed by transfecting HEK-293T cells (ATCC) with a Flag-PKR plasmid using a Fugene HD transfection reagent (Promega E2311), followed by cell lysis, after which immunoprecipitation was performed using Anti-FLAG M2 Affinity Gel (Sigma Aldrich A2220), and the PKR protein was eluted and purified using Pierce^{™} 3x DYKDDDDK Peptide (Thermo Fisher A36805). The purified PKR protein and IVT-synthesized hairpin RNAs were incubated at 4°C for 2 hours, followed by additional immunoprecipitation using a PKR antibody (Cell Signaling Technology 12297) to obtain PKR-RNA complexes. In addition, sequencing libraries were constructed for input RNA and PKR-RNA complexes, and RNA sequencing was then performed using a NovaSeq 6000 platform. Subsequently, sequencing reads were aligned using Hisat2, and the number of sequencing reads and fold changes between input RNA and PKR-bound RNA were calculated using FeatureCounts and DESeq2, thereby analyzing the binding affinity of each sdhRNA to PKR.

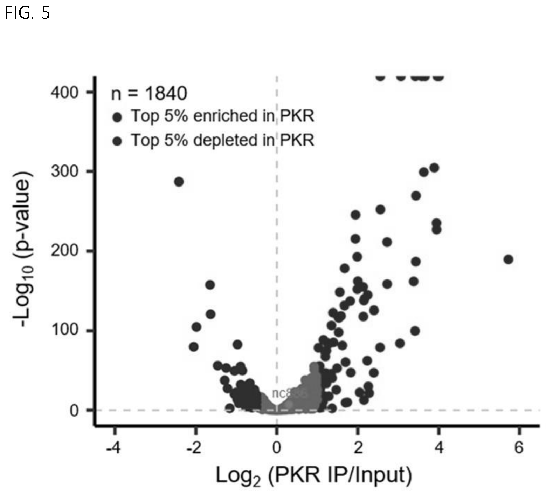

Therefore, sdhRNAs represented by SEQ ID NOs: 1 to 8 in Table 1 were obtained (FIG. 5).

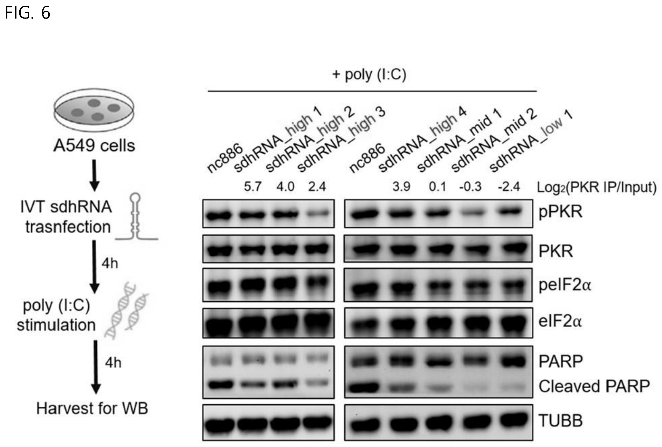

Among the sdhRNAs identified through the above screening and exhibiting varying binding affinities to PKR, seven sdhRNAs were evaluated for the ability to inhibit PKR activation. For this purpose, A549 cells were pre-treated with each sdhRNA at a concentration of 50 nM using a Lipofectamine 3000 transfection reagent (Invitrogen^{™} L3000015), followed by treatment, after 4 hours, with poly(I:C) (Sigma Aldrich P9582) at a concentration of 1000 ng/ml using a Lipofectamine 3000 transfection reagent (Invitrogen^{™} L3000015) to induce PKR activation. 4 hours after poly(I:C) treatment, the cells were lysed via sonication, and PKR activation was assessed by Western blotting (Experiment 4) (left panel of FIG. 6). As such, nc886 RNA, which has been reported to inhibit PKR activation, was used as a control.

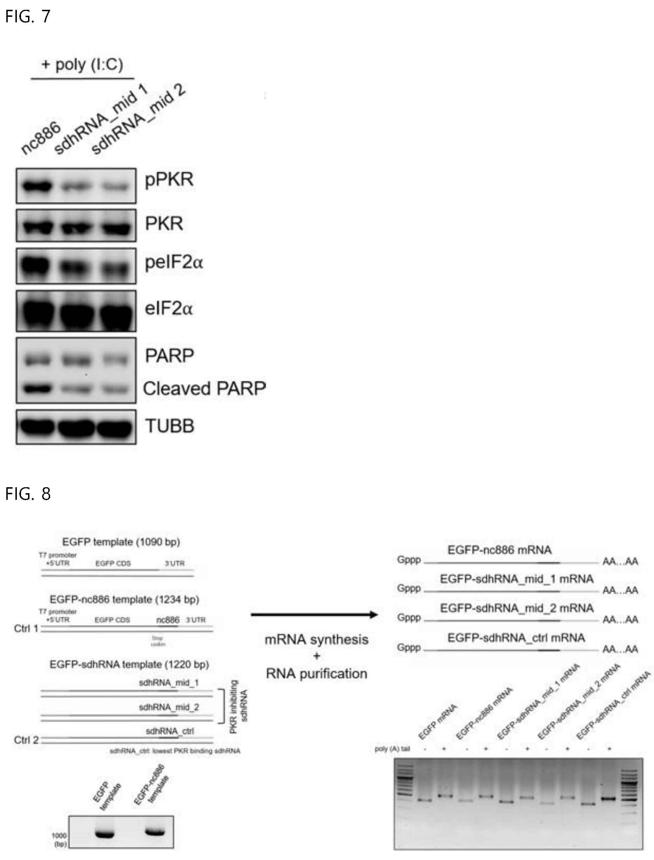

Therefore, compared to nc886, a marked reduction in PKR activation was observed in sdhRNA_high_3, sdhRNA _mid_1, sdhRNA_mid_2, and sdhRNA_low_1 (right panel of FIG. 6). Based on results of additional repeated experiments for mid_1 and mid_2 among the four identified sdhRNAs, a similarly pronounced decrease in PKR activation was confirmed for both sdhRNAs (FIG. 7).

### Example 5: Evaluation of antigen expression enhancement and modulation of innate immunity by EGFP mRNA incorporating sdhRNA structures

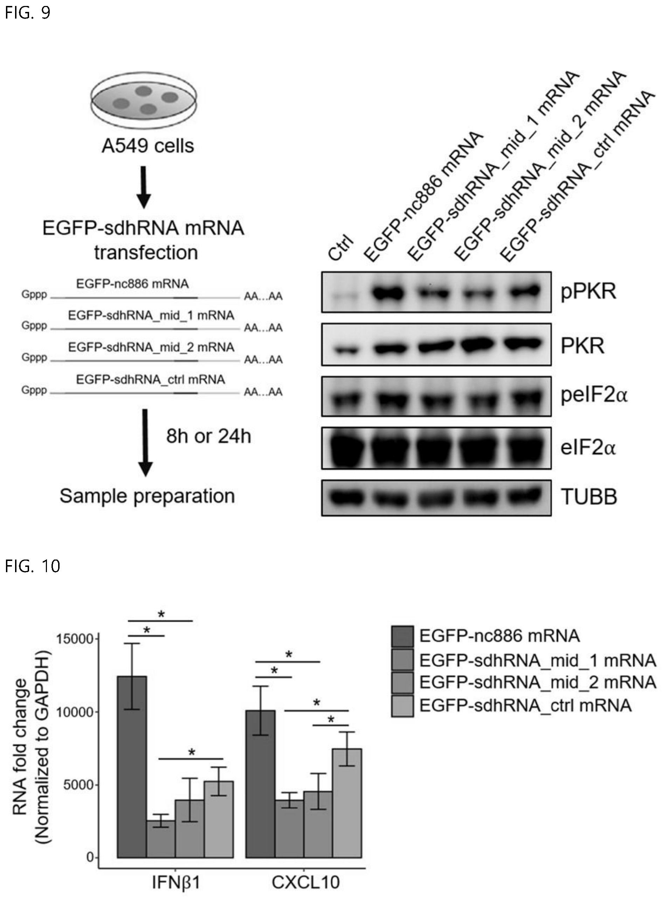

EGFP mRNA templates incorporating the sdhRNA sequences mid_1 and mid_2 obtained in Example 4 were synthesized through cloning (Experiment 3). As controls, nc886 and low_1 (sdhRNA_ctrl), which was expected not to bind to PKR, were used (FIG. 8). Subsequently, EGFP-nc886 mRNA, EGFP-sdhRNA_mid_1 mRNA, EGFP-sdhRNA_mid_2 mRNA, and EGFP-sdhRNA_ctrl mRNA were each synthesized (Experiment 2), and administered to A549 cells at a concentration of 400 ng/ml using a Lipofectamine 3000 transfection reagent (Invitrogen^{™} L3000015) (left panel of FIG. 9). After 24 hours, EGFP protein expression and innate immune responses were analyzed by Western blotting (Experiment 4) and RT-qPCR (Experiment 5).

Western blotting results confirmed that, compared to EGFP-nc886 mRNA and EGFP mRNA incorporating control sdhRNA, EGFP mRNA incorporating PKR-inhibitory sdhRNA exhibited the greatest reduction in PKR and eIF2α activation (right panel of FIG. 9). In addition, RT-qPCR results demonstrated that the expression levels of interferon-β and CXCL10, a representative ISG, were lowest in cells treated with EGFP mRNA incorporating PKR-inhibitory sdhRNA (FIG. 10).

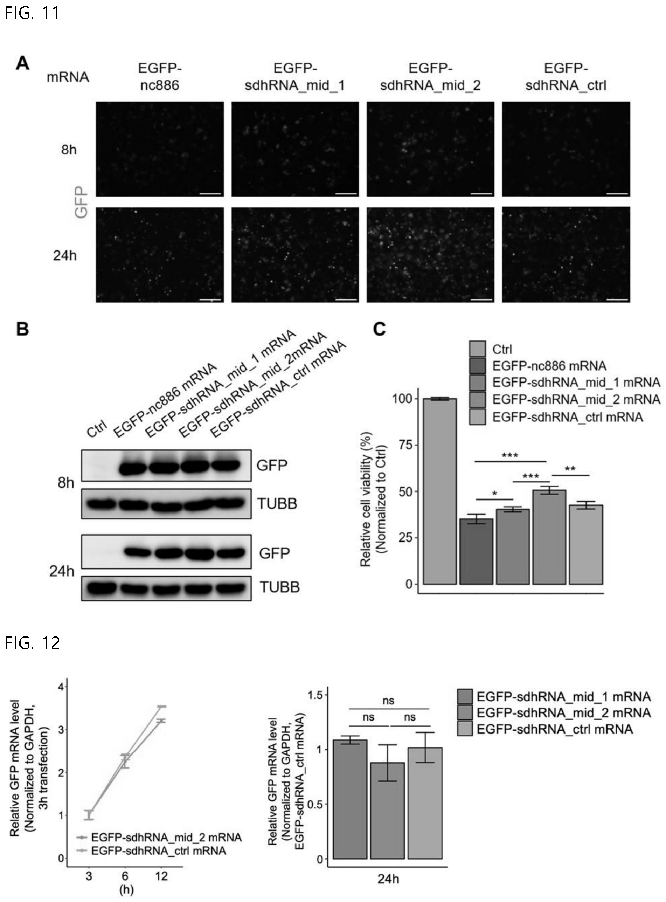

Consistent with fluorescence microscopy and Western blotting (Experiment 4), EGFP protein expression was also confirmed to be strongest in cells treated with EGFP mRNA incorporating PKR-inhibitory sdhRNA, as compared to EGFP-nc886 mRNA and EGFP mRNA incorporating control sdhRNA (FIGs. 11A and 11B). Furthermore, when cytotoxicity induced by mRNA administration was evaluated using a CCK-8 assay kit (Dojindo CK04) according to the manufacturer's instructions, EGFP mRNA incorporating PKR-inhibitory sdhRNA exhibited the lowest level of cytotoxicity (FIG. 11C).

To determine whether these effects were attributable to changes in cellular uptake efficiency or stability of mRNA depending on the incorporation of sdhRNA, EGFP mRNA was administered to A549 cells using a Lipofectamine 3000 transfection reagent (Invitrogen^{™} L3000015), and the intracellular expression levels of EGFP mRNA over time were analyzed by RT-qPCR (Experiment 5). Therefore, it was confirmed that the incorporation of sdhRNA did not significantly affect the cellular uptake efficiency or stability of EGFP mRNA (FIG. 12).

### Example 6: Evaluation of lipid nanoparticle (LNP)-based delivery of EGFP mRNA incorporating sdhRNA

Since lipid nanoparticles (LNPs), rather than lipofectamine, are employed for *in vivo* administration in animals/humans, whether administration of sdhRNA-incorporating mRNA to cells via LNPs would exhibit a similar level of modulation of innate immunity as observed in the above experimental example was evaluated. The LNPs used in this example were composed of (6Z,9Z,28Z,31Z)-heptatriaconta-6,9,28,31-tetraen-19-yl 4-(dimethylamino)butanoate, 1,2-distearoyl-sn-glycero-3-phosphocholine, cholesterol, and 1,2-dimyristoyl-rac-glycero-3-methoxypolyethylene glycol-2000 at a molar ratio of 50:10:38.5:1.5. The sdhRNA-incorporating EGFP mRNA and LNPs were mixed at a mass ratio of 1:40 using an ethanol mixing method, followed by buffer exchange to PBS to prepare a stable mRNA-LNP complex. Thereafter, the complex was administered to A549 cells at an mRNA concentration of 1000 ng/ml, and after 24 hours, EGFP protein expression levels were compared by Western blotting (Experiment 4). Therefore, LNP-based delivery exhibited higher efficiency than lipofectamine, and also, cytotoxicity analysis using a CCK-8 assay kit (Dojindo CK04) confirmed that the LNP-based delivery exhibited low cytotoxicity.

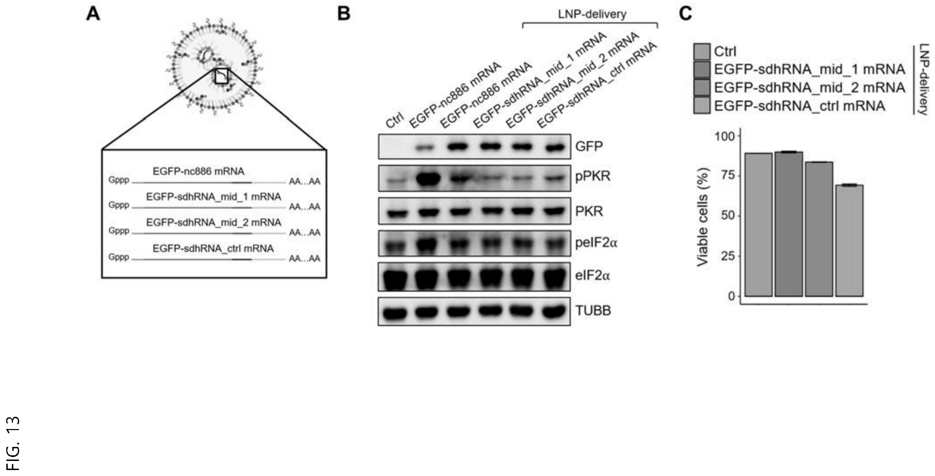

Specifically, analysis of PKR activation confirmed that PKR activation was markedly reduced when LNPs were used (FIG. 13B), and for EGFP mRNA incorporating PKR-inhibitory sdhRNA, PKR activation was almost completely suppressed and cell death was minimal (FIG. 13C). These results demonstrated that delivery of sdhRNA-incorporating mRNA into cells via LNPs could enhance antigen expression while suppressing innate immune responses.

### Example 7: Analysis of immunoregulatory effects of sdhRNA in mRNA containing pseudouridine

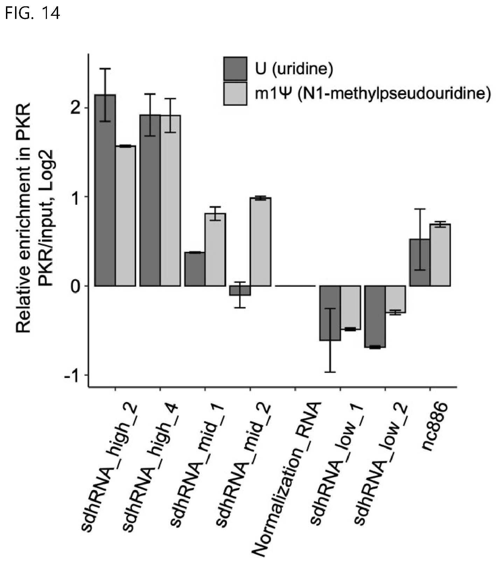

Additional immunoregulatory effects on innate immunity were evaluated upon incorporation of sdhRNA into the 3' UTR of mRNA containing pseudouridine-modified nucleotides, which are widely used in current mRNA vaccine technologies. First, to analyze the effect of pseudouridine incorporation on the binding affinity between sdhRNA and PKR, RNA in which uridine was substituted with pseudouridine (N1-Me-pUTP, GLPBIO GB20016) was synthesized for the eight sdhRNAs used in the above experimental example (Experiment 2). The synthesized sdhRNAs were incubated with PKR protein at 4°C for 2 hours, followed by immunoprecipitation using a PKR antibody (Cell Signaling Technology 12297) to obtain PKR-bound sdhRNAs. Subsequently, binding affinity to PKR was analyzed by comparing input RNA and PKR-bound RNA using RT-qPCR (Experiment 5). For most sdhRNAs, it was confirmed that pseudouridine-incorporating sdhRNAs exhibited a similar level of binding affinity to PKR (FIG. 14).

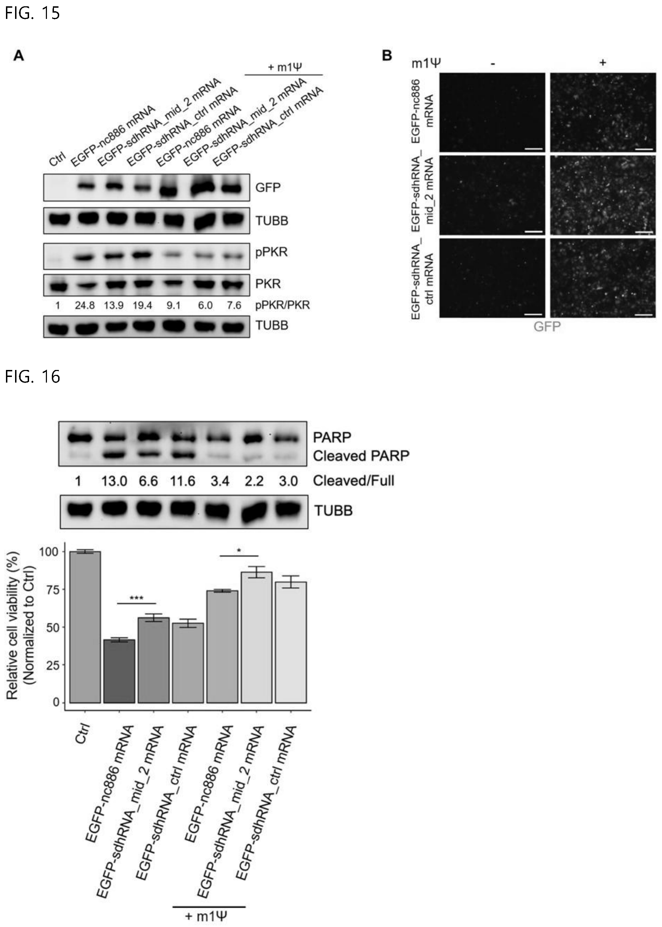

EGFP mRNA incorporating a PKR-inhibitory sdhRNA (sdhRNA _mid_2) into the 3' UTR was synthesized with simultaneous incorporation of pseudouridine and sdhRNA (Experiment 2), and then administered to A549 cells at a concentration of 400 ng/ml using a Lipofectamine 3000 transfection reagent (Invitrogen^{™} L3000015) to analyze effects on antigen expression and immune activation. First, fluorescence microscopy and Western blotting (Experiment 4) confirmed that incorporation of pseudouridine enhanced EGFP mRNA antigen expression and suppressed PKR activation (FIGs. 15A and 15B). Furthermore, when sdhRNA was incorporated into the 3' UTR of mRNA containing pseudouridine-modified nucleotides, further enhanced antigen expression and reduced PKR activation were observed. In addition, as confirmed in the above experimental example, incorporation of sdhRNA was associated with reduced mRNA-induced cell death. Western blotting (Experiment 4) further showed that the level of cleaved PARP, a major apoptosis marker, was lowest in cells treated with mRNA incorporating both pseudouridine and sdhRNA. Consistently, cytotoxicity analysis using a CCK-8 assay kit (Dojindo CK04) demonstrated high cell viability (FIG. 16).

### Example 8: Evaluation of antigen production enhancement by incorporating sdhRNA into SARS-CoV-2 Spike mRNA

To assess the applicability of the PKR inhibitory effect of the developed sdhRNA to mRNA vaccines, using a plasmid capable of encoding the SARS-CoV-2 Spike protein, Spike mRNA was synthesized after incorporating nc886 as a control and sdhRNA_mid_2, which showed the best efficacy as the experimental group, into the 3' UTR (Experiments 1, 2, and 3).

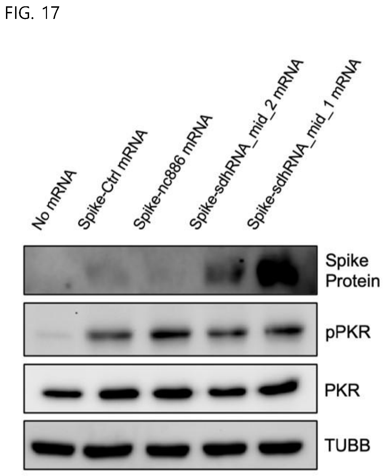

The synthesized Spike mRNA was administered to A549 cells at a concentration of 1000 ng/ml using a TransIT-mRNA transfection kit (Mirus MIR2250), and after 24 hours, Spike antigen expression and PKR activation were evaluated by Western blotting (Experiment 4). Therefore, it was confirmed that incorporation of sdhRNA into the Spike mRNA increased Spike protein expression and suppressed PKR activation (FIG. 17).

### Example 9: Analysis of in vivo efficacy of SARS-CoV-2 Spike mRNA incorporating sdhRNA motif

The present inventors encapsulated SARS-CoV-2 Spike mRNA incorporating the constructed sdhRNA in the 3' UTR into the prepared LNPs using a NanoAssemblr Benchtop Instrument (Precision Nanosystems Inc.) at a final LNP:RNA weight ratio of 10:1, and administered the resulting formulation to a mouse model (Samtako Korea) to evaluate immunogenicity. Specifically, Balb/c mice (4 weeks old, female) were intramuscularly injected (I.M.) twice with an mRNA vaccine at a dose of 5 µg at 3-week intervals. Two weeks after the final immunization, serum and splenocytes were collected to assess cellular immunogenicity and binding antibody titers. In the mouse study, mRNA incorporating pseudouridine-modified nucleotides was used, and nc886, mid_1, and mid_2 were employed as sdhRNAs.

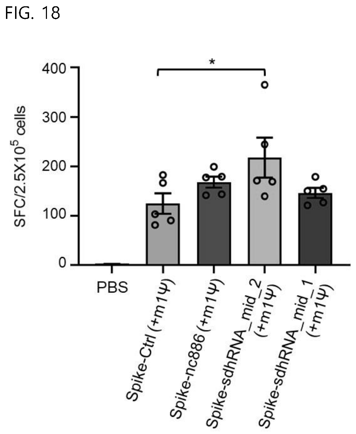

To evaluate whether the produced SARS-CoV-2 mRNA vaccine induces a cellular immune response, an ELISpot assay for measuring T cells secreting virus-specific IFN-γ from the splenocytes of immunized mice was performed. Specifically, spleens were isolated from mice immunized with a pan-SARS-CoV-2 mRNA vaccine in RPMI medium, and the spleens were dissociated into single cells using a gentleMACS instrument. The number of splenocytes was determined using a LUNA cell counter. Splenocytes were seeded in a 96-well plate of a Mouse IFN-γ ELISpot Plus Kit (XEL 485, R&D SYSTEMS) at 2.5x10⁵ cells/well, and as a stimulus, SARS-CoV-2 peptides were diluted to a final concentration of 1 µg/well and added to the corresponding wells. The cell-stimulus mixture was incubated at 37°C in 5% CO₂ for 12 to 48 hours, followed by a series of processes including removal of the cell-stimulus mixture, washing with PBS, incubation with a primary antibody for 2 hours, washing with PBS, incubation with an ALP-conjugated secondary antibody for 1 hour, addition of a substrate solution (BCIP/NBT-plus), reaction until spot formation, and counting of the spots using CTL automated equipment. Analysis of the number of T cells secreting virus-specific IFN-γ indicated that, compared with the two control groups, sdhRNA_mid_2 induced a superior cellular immune response (FIG. 18).

To evaluate whether the produced SARS-CoV-2 mRNA vaccine elicits Spike antigen-specific antibody titers, ELISA was performed using immunized mouse sera. Individual sera were initially diluted 1:100 and then subjected to 3-fold serial dilutions in 3% skim milk, and added to wells coated with SARS-CoV-2 Spike Protein (S1+S2 ECD, 40589-V08B1, Sino Biological) at 1 µg/ml, followed by standard ELISA procedures (primary antibody incubation, washing, secondary antibody incubation, washing, and TMB solution incubation and reaction). Finally, the reaction was terminated with a stop solution (2M H₂SO₄), and optical density (OD) was measured at 450nm using a microplate reader.

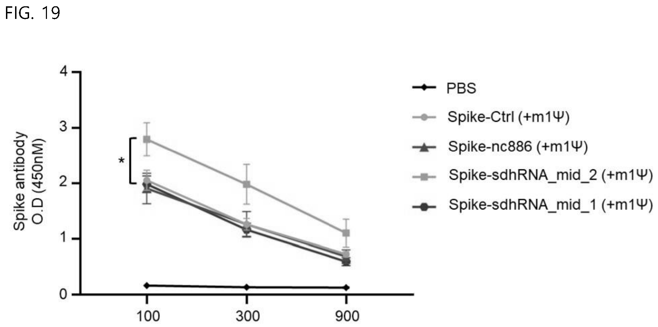

Analysis of Spike antigen-specific antibodies titers showed that, compared to the two control groups, sdhRNA_mid_2 exhibited high binding antibody titers (FIG. 19).

### [Industrial Applicability]

The sdhRNA motif according to the present invention binds to protein kinase R (PKR), which is activated upon recognition of RNA, thereby inhibiting PKR activation. Accordingly, when administered to cells in combination with exogenous RNA or as an insert in the 3'-UTR of mRNA, the sdhRNA motif is effective at reducing RNA-induced PKR activation, alleviating RNA-induced cell death, and decreasing expression of interferon-stimulated genes (ISGs), as well as improving the expression efficiency of a protein encoded by the mRNA. Therefore, the sdhRNA motif is useful for various RNA-based applications, including vaccines and *in vivo*/*ex vivo* gene therapeutics.

Having described certain parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

### [Sequence List Free Text]

An electronic file is attached.

## Claims

1. A short duplex hairpin nucleic acid (sdhNA) represented by a nucleotide sequence of any one of SEQ ID NOs: 1 to 8, which suppresses an innate immune response.

2. A nucleic acid construct comprising, in a 5' to 3' direction:
a) at least one coding region;
b) a 3'-untranslated region (3'-UTR);
c) the short duplex hairpin nucleic acid according to claim 1; and
d) a poly(A) tail of 10 to 1000 nucleotides or a poly(A) tail-like sequence.

3. The nucleic acid construct according to claim 2, further comprising a 5'-cap structure and a 5'-UTR polynucleotide upstream of the at least one coding region.

4. The nucleic acid construct according to claim 3, wherein the 5'-cap structure is selected from the group consisting of m⁷GpppAₘpG, m7,3'-OmeApppG, m7GpppA, and m₂^{7,3'-O}G(5')ppp(5')G (ARCA) .

5. The nucleic acid construct according to claim 3, wherein the 5'-UTR polynucleotide is selected from the group consisting of an α-globin 5'-UTR; an Hsp70 5'-UTR; an axon dynein heavy chain 2 (DNAH2) 5'-UTR; a hydroxysteroid dehydrogenase (3β-HSD) 5'-UTR; a 5'-UTR represented by a nucleotide sequence of SEQ ID NO: 11; and a 5'-UTR represented by a nucleotide sequence of SEQ ID NO: 12.

6. The nucleic acid construct according to claim 2, wherein the coding region encodes at least one protein selected from the group consisting of an antigenic protein, an allergenic protein, a therapeutic protein, and fragments, variants, or derivatives thereof.

7. The nucleic acid construct according to claim 6, wherein the antigenic protein is at least one selected from the group consisting of a tumor antigen, a pathogenic antigen, an autoantigen, an alloantigen, and an allergenic antigen.

8. The nucleic acid construct according to claim 7, wherein the antigen is selected from the group consisting of EGFP and a Spike protein.

9. The nucleic acid construct according to claim 7, wherein the pathogenic antigen is selected from the group consisting of bacterial, viral, fungal, and protozoan antigens.

10. The nucleic acid construct according to claim 9, wherein the virus is a coronavirus.

11. The nucleic acid construct according to claim 1, wherein the poly(A) tail-like sequence comprises at least one nucleotide other than adenine selected from the group consisting of uracil (U), cytosine (C), and guanine (G), inserted between multiple adenines or at an end of the poly(A) tail.

12. The short duplex hairpin nucleic acid according to claim 1, wherein the short duplex hairpin nucleic acid is RNA.

13. The nucleic acid construct according to claim 2, wherein the nucleic acid construct is selected from the group consisting of mRNA, viral RNA, self-amplifying RNA, and replicon RNA.

14. The nucleic acid construct according to claim 2, wherein the nucleic acid construct comprises at least one backbone-modified, sugar-modified, or base-modified nucleic acid.

15. The nucleic acid construct according to claim 14, wherein the base-modified nucleic acid is selected from the group consisting of pseudouridine (Ψ), N1-methylpseudouridine (m1Ψ), 5-methyluridine (m5U), 2-thiouridine (s2U), 2'-O-methyluridine (Um), 5-methylcytidine (m5C), and 5-methoxyuridine (5moU).

16. The nucleic acid construct according to claim 2, wherein the 3'-UTR is selected from the group consisting of a β-globin 3'-UTR; a CYBA 3'-UTR; an albumin 3'-UTR; a growth hormone (GH) 3'-UTR; a VEEV 3'-UTR; a hepatitis B virus (HBV) 3'-UTR; an α-globin 3'-UTR; a DEN 3'-UTR; a PAV barley yellow dwarf virus (BYDV-PAV) 3'-UTR; an elongation factor 1 alpha 1 (EEF1A1) 3'-UTR; a manganese superoxide dismutase (MnSOD) 3'-UTR; a β-subunit of mitochondrial H(+)-ATP synthase (β-mRNA) 3'-UTR; a GLUT1 3'-UTR; a MEF2A 3'-UTR; a β-F1-ATPase 3'-UTR; a 3'-UTR represented by a nucleotide sequence of SEQ ID NO: 13; and a 3'-UTR represented by a nucleotide sequence of SEQ ID NO: 14.

17. A vaccine composition comprising the nucleic acid construct according to any one of claims 2 to 16.

18. The vaccine composition according to claim 17, wherein the nucleic acid construct is complexed with at least one lipid to form a lipid nanoparticle or a liposome.

19. The vaccine composition according to claim 18, further comprising at least one adjuvant or active agent.
